# EUROPÄISCHE PATENTSCHRIFT

(11) **EP 4 038 620 B1**
(45) Veröffentlichungstag und Bekanntmachung des Hinweises auf die Patenterteilung: **05.07.2023**
(21) Anmeldenummer: 20764411.3
(22) Anmeldetag: 04.09.2020
(51) Int. Cl.: G16C 60/00, G16C 20/70

(54) **VERFAHREN ZUR GENERIERUNG THERMOPLASTISCHER ZUSAMMENSETZUNGEN FÜR MECHANISCH UND/ODER THERMISCH BELASTETE BAUTEILE**
METHOD FOR PRODUCING THERMOPLASTIC COMPOSITIONS FOR MECHANICALLY AND / OR THERMALLY LOADED COMPONENTS
PROCÉDÉ DE GÉNÉRATION DE COMPOSITIONS THERMOPLASTIQUES POUR COMPOSANTS À CHARGE MÉCANIQUE ET / OU THERMIQUE

(30) Priorität: 01.10.2019 EP 19200877
(43) Veröffentlichungstag der Anmeldung: 10.08.2022
(73) Patentinhaber: Evonik Operations GmbH, 45128 Essen (DE)
(72) Erfinder: STACHE, Wiebke, 45699 Herten (DE); WEINELT, Frank, 48727 Billerbeck (DE)
(74) Vertreter: Evonik Patent Association
(86) Internationale Anmeldenummer: PCT/EP2020/074747
(87) Internationale Veröffentlichungsnummer: WO 2021/063630

(56) Entgegenhaltungen:
- WO-A1-99/07779
- CN-A- 106 777 922
- KR-A- 20190 007 770
- IVAN KOPAL ET AL: "Modeling the Temperature Dependence of Dynamic Mechanical Properties and Visco-Elastic Behavior of Thermoplastic Polyurethane Using Artificial Neural Network", POLYMERS, Bd. 9, Nr. 12, 18. Oktober 2017 (2017-10-18), Seite 519, XP055702470, DOI: 10.3390/polym9100519

## Beschreibung

Die Erfindung betrifft ein Verfahren zur Generierung thermoplastischer Zusammensetzungen für mechanisch und/oder thermisch belastete Bauteile.

Zusammensetzungen für mechanisch und/oder thermisch belastete Bauteile, sind komplexe Mischungen von Rohstoffen. Übliche Zusammensetzungen oder Rezepturen bzw. Compounds für mechanisch und/oder thermisch belastete Bauteile enthalten etwa 20 Rohstoffe, im Folgenden auch "Komponenten" genannt. Diese Zusammensetzungen bestehen beispielsweise aus Rohstoffen die ausgewählt sind aus Polymeren, Verarbeitungshilfsmitteln, Stabilisatoren, Farbmitteln, Leitfähigkeitshilfsmittel (elektrisch), thermische Leitfähigkeitsadditive, Verstärkungsmittel, Schlagzähmodifikatoren; Füllstoffe; Flammschutzmittel und Weichmacher.

Bisher werden neue Zusammensetzungen und Compounds mit bestimmten, gewünschten Eigenschaften anhand von Erfahrungswerten spezifiziert und danach hergestellt und getestet. Die Komposition einer neuen Zusammensetzung, die bestimmte Erwartungen an deren chemischen, physikalischen, optischen, haptischen und sonstigen messtechnisch erfassbaren Eigenschaften erfüllt, ist aufgrund der Komplexität der Wechselwirkungen auch für einen Fachmann kaum vorhersehbar. Durch die Mannigfaltigkeit der Wechselwirkungen der Rohstoffe untereinander und damit einhergehend einer Vielzahl von Fehlversuchen ist dieses Vorgehen sowohl zeit- als auch kostenintensiv. Gegebenenfalls sind die Eigenschaften bei gleicher Komposition der Zusammensetzung auch von den Herstellungsbedingungen abhängig.

Aus der US 2018/0276348 A1 ist ein kognitives Computersystem zur Herstellung chemischer Formulierungen bekannt. Das System bestimmt eine chemische Formulierung die bestimmte Einschränkungen erfüllt, produziert und testet die chemische Formulierung. Dieses Computersystem beruht auf dem Training eines lernenden Systems mit vorhandenen Daten chemischer Formulierungen. Die Erstellung hinreichend großer Datensätze um eine Lern-Logik auf diesen zu trainieren ist jedoch sehr aufwändig und aufgrund des hohen Zeit- und Materialverbrauchs auch teuer. In vielen Fällen ist es auch nicht möglich, einfach auf einen in den meisten Labors vorhandenen Datensatz bereits hergestellter und analysierter Zusammensetzungen zurückzugreifen. Dafür kann es verschiedene Gründe geben: das Labor wurde frisch eingerichtet und verfügt noch nicht über eine entsprechende Datenbasis. Das Labor etabliert eine neue Produktlinie und hat noch keine Erfahrung und entsprechende Datensätze betreffend die Eigenschaften dieser neuen Produktlinie. Oder es sind zwar Daten vorhanden, diese sind jedoch vom Umfang her zu gering oder von ihrer historisch bedingten Zusammensetzung her zu unausgewogen (gebiassed"), um als Trainingsdatensatz verwendbar zu sein.

KR 2019/0007770 A beschreibt ein Verfahren zur Ermittlung einer thermoplastischen Zusammensetzung für mechanisch belastete Bauteile, wobei die Zusammensetzung mit Hilfe statistischer Methoden durch ein Computersystem generiert wird, wobei das Computersystem auf eine Datenbank Zugriff hat, wobei in der Datenbank bekannte Zusammensetzungen mit deren Komponenten und Eigenschaften gespeichert sind, und wobei das Computersystem ein neuronales Netzwerk umfasst. Daten von Materialanalysen, insbesondere NMR, GPC und DSC Daten dienen zur Vorhersage von neuen Zusammensetzungen, welche aufgrund der berechneten Rezeptur hergestellt werden können.

Somit sind sowohl der von einem Menschen durchgeführten als auch der computergestützten Einschätzung und Prognose bezüglich der Komponenten einer Zusammensetzung mit gewünschten Eigenschaften derzeit sehr enge Grenzen gesetzt. Dies gilt insbesondere für komplexe Zusammensetzungen mit vielen relevanten Eigenschaften und vielen Komponenten, wie dies bei thermoplastischen Zusammensetzungen für mechanisch und/oder thermisch belastete Bauteile der Fall ist, da die Komponenten auf komplexe Art miteinander wechselwirken und die Eigenschaften der entsprechenden chemischen Produkte bestimmen.

Deshalb müssen derzeit neue Zusammensetzungen zunächst chemisch real hergestellt und deren Eigenschaften dann gemessen werden, um abschätzen zu können, ob die Zusammensetzungen bestimmte erforderliche Eigenschaften aufweisen. Zwar gibt es schon Ansätze zur automatischen Vorhersage von Eigenschaften chemischer Substanzen, die Erstellung eines Trainingsdatensatzes hinreichender Größe und Qualität ist aber oft noch aufwändiger als die in Frage kommende Zusammensetzung direkt herzustellen und zu testen. Die Entwicklung neuer thermoplastischer Zusammensetzungen für mechanisch und/oder thermisch belastete Bauteile ist besonders aufwändig und erfordert viel Zeit.

Es ist daher die Aufgabe der vorliegenden Erfindung, ein Verfahren bereitzustellen, durch welches die Entwicklung einer neuen Zusammensetzung oder die Entwicklung eines Compounds einfacher, zeitsparender und kostengünstiger erreicht wird.

Die Aufgabe wird durch das Verfahren zur Generierung thermoplastischer Zusammensetzungen für mechanisch und/oder thermisch belastete Bauteile gemäß Anspruch 1 sowie ein entsprechendes Computersystem und Computerprogrammprodukt gelöst. Ausführungsformen der Erfindung sind in den abhängigen Ansprüchen angegeben. Ausführungsformen der vorliegenden Erfindung können frei miteinander kombiniert werden, wenn sie sich nicht gegenseitig ausschließen.

Die Belastung der beanspruchten Bauteile umfasst weiterhin auch Belastungen durch Chemikalien und Witterung.

In einem Aspekt betrifft die Erfindung ein Verfahren zur Generierung thermoplastischer Zusammensetzungen für mechanisch und/oder thermisch belastete Bauteile. Die Zusammensetzung wird durch ein Computersystem generiert. Das Computersystem hat auf eine Datenbank Zugriff, in welcher bekannte Zusammensetzungen mit deren Komponenten und Eigenschaften gespeichert sind. Das Computersystem ist mit einer Anlage zur Herstellung und Prüfung thermoplastischer Zusammensetzungen verbunden.

Das Computersystem beinhaltet ein neuronales Netzwerk (Neural Network) und ein Active Learning Modul. Das Verfahren umfasst folgende Schritte:
a. Verwendung von in der Datenbank gespeicherten bekannten Zusammensetzungen zum Trainieren des neuronalen Netzwerks, wobei zum Training eine Loss-Funktion minimiert wird,
b. Prüfung, ob der Wert der Loss-Funktion ein vorgegebenes Kriterium erfüllt, wobei selektiv für den Fall, dass das Kriterium nicht erfüllt ist, die folgenden Schritte durchgeführt werden:
   i. Auswahl einer Versuchs-Zusammensetzung aus einer Menge vorgegebener Versuchs-Zusammensetzungen durch das Active Learning Modul,
   ii. Veranlassung der Anlage durch das Computersystem zur Herstellung und Prüfung der ausgewählten Versuchs-Zusammensetzung,
   iii. Trainieren des neuronalen Netzwerks unter Verwendung der ausgewählten Versuchs-Zusammensetzung und deren von der Anlage erfassten Eigenschaften,
   iv. Wiederholte Durchführung von Schritt b.
c. Generierung einer Prognosezusammensetzung für mechanisch und/oder thermisch belastete Bauteile durch Eingabe eines Eingabevektors in das neuronale Netzwerk,
d. Ausgabe der Prognosezusammensetzung durch das neuronale Netzwerk.

Das neuronale Netz kann zum Beispiel ein Multi-Layer Perceptron (MLP), ein Convolutional Neural Network (CNN), ein Recurrent Neural Network (RNN), Memory-Augmented Neural Network (MANN) und Tree-Recursive Neural Network sein oder auch kombinierte Methoden (Hybrid/Complex Models) vereinen wie zum Beispiel Generative Adversarial Networks (GAN), Named Entity Recognition (NER) und/oder Deep Reinforcement Learning.

Bevorzugt enthält das neuronale Netz ein rekurrentes Netz, ein faltungsneronales Netz oder die Kombination aus beiden; mehr bevorzugt ist das neuronale Netz ein rekurrentes Netz, ein faltungsneronales Netz oder die Kombination aus beiden.

Dies ist vorteilhaft, da in einem voll- oder semi-automatischen iterativen Verfahren ein existierender Trainingsdatensatz schrittweise und gezielt um aussagekräftige weitere Trainingsdaten in Form von Versuchs-Zusammensetzungen und deren empirisch ermittelten Eigenschaften erweitert werden, wobei bei jeder Iteration ein erneutes Training des neuronalen Netzes auf dem erweiterten Trainingsdatensatz erfolgt, wodurch die Vorhersagequalität des Netzes nach jeder Iteration verbessert wird, bis die Loss-Funktion das Kriterium erfüllt, also ein Prognosefehler des Netzwerks hinreichend klein geworden ist.

Aufgrund der begrenzten Zahl bereits bekannter Zusammensetzungen ist das nach der initialen Trainingsphase erhaltene trainierte neuronale Netz oftmals noch nicht in der Lage, eine Prognosezusammensetzung verlässlich vorherzusagen, die eine Reihe gewünschter Eigenschaften aufweist. Hierzu ist die verwendete Datenbasis oftmals zu klein.

Eine Erweiterung des Trainingsdatensatzes durch ein Active Learning Modul erlaubt es, gezielt einige wenige Versuchs-Zusammensetzungen auszuwählen, von welchen die Qualität des neuronalen Netzes und seiner Vorhersagen besonders profitiert. In mehreren Iterationen können somit durch automatische und durch gezielte Auswahl von Versuchs-Zusammensetzungen, die eine besonders starke Verbesserung des prädiktiven Modells des neuronalen Netzes versprechen, sowie automatisch oder manuell ausgeführte Synthese und Analyseschritte auf Basis der jeweils ausgewählten Versuchs-Zusammensetzung, die Vorhersagekraft des neuronalen Netzes auf effiziente Weise schnell verbessert werden. Wenn nach einer Iteration festgestellt wird, dass die Loss-Funktion das Kriterium erfüllt, ist eine Erweiterung des Trainingsdatensatzes nicht mehr nötig, da die Vorhersagekraft des trainierten neuronalen Netzwerks als ausreichend angesehen werden kann.

In einem weiteren vorteilhaften Aspekt kann das Active Learning Modul durch automatische Auswahl von Versuchs-Zusammensetzungen, die die Vorhersagekraft des neuronalen Netzwerks besonders steigern, bestehende Datenbestände bekannter Zusammensetzungen so zu erweitern, dass eine Unbalanciertheit der Trainingsdaten weitgehend automatisch ausgeglichen werden kann.

Nach Ausführungsformen der Erfindung wird die Menge der Versuchs-Zusammensetzungen durch ein Versuchsplanungsprogramm automatisch erzeugt. Beispielsweise können die Versuchs-Zusammensetzungen auf Basis der bekannten Zusammensetzungen durch Hinzufügen oder Weglassen von Komponenten oder durch die Modifikation der Menge bzw. Konzentration einer oder mehrerer Komponenten automatisch erzeugt werden.

Dies kann vorteilhaft sein, da eine sehr große Anzahl an Versuchs-Zusammensetzungen automatisch erzeugt werden kann. Es kann somit ein großer Datenraum mit Kandidaten-Zusammensetzungen aufgespannt werden. Der von den Versuchs-Zusammensetzungen aufgespannte Datenraum kann insbesondere den Vorteil haben, dass dieser weniger "gebiassed" ist, wenn z. B. das Versuchsplanungsprogramm dazu ausgebildet ist, die Versuchs-Zusammensetzungen so zu erzeugen, dass im Wesentlichen jede Komponente ähnlichen und einen breiten Datenraum abdeckenden Variationen (Entfernen, Hinzufügen, Konzentrationsänderung) unterworfen wird um die Versuchs-Zusammensetzungen zu generieren.

Thermoplasten sind Kunststoffe, die sich in einem bestimmten Temperaturbereich (thermoplastisch) verformen lassen. Dieser Vorgang ist reversibel, das heißt, er kann durch Abkühlung und Wiedererwärmung bis in den schmelzflüssigen Zustand beliebig oft wiederholt werden

Nach Ausführungsformen der Erfindung sind die Komponenten der bekannten Zusammensetzungen und/oder der Versuchs-Zusammensetzungen bestehend aus einem oder mehreren Polymeren wie Polyamid, Polyester, Polyolefine, Polycarbonate und Polyaryletherketone; sowie optional weiteren Komponenten wie Verarbeitungshilfsmittel wie Gleitmittel, Fließhilfen, Nukleierungsmittel und Entformungshilfen; Stabilisatoren wie Hitzestabilisatoren, Antioxydantien, Lichtstabilisatoren und UV-Absorber; Farbmittel wie Farbstoffe, Pigmente und optische Aufheller; Leitfähigkeitshilfsmittel (elektrisch) wie Leitfähigkeitsruße, Carbo-Nano-Tubes, Graphen, ionische Flüssigkeiten oder Komplexsalze; thermische Leitfähigkeitsadditive wie Aluminiumoxid oder Bornitrid; Verstärkungsmittel wie Glasfasern und Carbonfasern; Schlagzähmodifikatoren; Füllstoffe; Flammschutzmittel und Weichmacher.

Nach Ausführungsform umfasst die Herstellung gegebenenfalls die chemische Synthese der Komponenten wie die der Polymere, sowie die Herstellung der Zusammensetzungen aus den Komponenten.

Nach Ausführungsformen der Erfindung sind die optionalen Herstellungsmöglichkeiten ausgewählt aus der Gruppe bestehend aus Extrusion und Spritzguss wobei die Herstellungsparameter ausgewählt sind aus Maschinenangaben, Zylindertemperaturen, Einspritzprofil, Umschaltweg, Umschaltdruck (hydr.), Forminnendruck beim Umschalten, Nachdruckprofil, Nachdruckzeit, Nachdruck (hydr.), Plastifizierung, Dosierhub (vPLAST), Dosiergeschwindigkeit, Dosierhub (pSTAU), Staudruck (hydr.), Restmassepolster, Restkühlzeit, Max. Forminnendruck pN, Dosierzeit, Dosierverzögerungszeit, Entlastung nach Dosieren, Entlastungsgeschwindigkeit, Dosierhub (Ist), Zykluszeit, Dosierhub, Schließkraft, Temperatur der Einzugszone, Drehmoment, Schneckenkonfiguration, Druck, Drehmomentmaximum, Schmelzefiltration, Entgasung, Nachtrocknungstemperatur, Ansatzmenge, Zylindertemperatur, Durchsatz, Massetemperatur, Düsentemperatur, Vakuumentgasung, Nachtrocknungsdauer, Nachtrocknungsatmosphäre, Zylinderzone, Drehzahl.

Nach Ausführungsformen der Erfindung sind die von der Anlage erfassten Eigenschaften der Versuchs-Zusammensetzung (Compounds) ausgewählt aus der Gruppe bestehend aus Kerbschlagzähigkeit, Berstumfangsspannung, BET-Oberfläche, Aminoendgruppengehalt, Carboxylendgruppengehalt, Dichte, Dielektrizitätszahl, Konventionelle Durchbiegung, Versagensart, Streckspannung, Durchgangwiderstand, Maximalkraft-Durchstoss, Anzahl Brüche - Fallhammer- und Kugelfalltests, Farbzahl Lab, Auszugskraft (Fittings), Korrigierte inhärente Viskosität (CIV), Permeation (Zeit), Schmelze-Volumenfließrate (MVR), Sauerstoff-Index, Oberflächenwiderstand, Schmelzeviskosität, Endgruppensumme, Schmelztemperatur, Schmelzenthalpie, Glasübergangstemperatur, Kristallinität, Maßänderung, Stampfdichte, Transmission, Brennbarkeit (UL94), Vicat-Erweichungstemperatur, Formbeständigkeitstemperatur und Wassergehalt, Bruchspannung, Bruchdehnung, E-Modul, Wärmebeständigkeit.

Nach Ausführungsformen der Erfindung erfolgt die Ausgabe der Prognosezusammensetzung auf einem Nutzerinterface des Computersystems. Bei dem Nutzer-Interface kann es sich zum Beispiel um einen Bildschirm, einen Lautsprecher und/oder einen Drucker handeln.

Dies kann vorteilhaft sein, da der Nutzer die Prognosezusammensetzung vor deren Übermittlung an die chemische Anlage zum Zwecke der Herstellung noch einmal manuell auf Plausibilität prüfen kann.

Nach Ausführungsformen weist die Anlage mindestens zwei Bearbeitungsstationen auf.

Nach Ausführungsformen umfasst das Verfahren ferner: Eingabe einer Zusammensetzung an einen Prozessor, der die Anlage steuert, wobei die in den Prozessor eingegebene Zusammensetzung die ausgewählte Versuchs-Zusammensetzung oder die Prognosezusammensetzung ist, wobei der Prozessor die Anlage ansteuert, die eingegebene Zusammensetzung herzustellen, wobei in den mindestens zwei Bearbeitungsstationen die Herstellung der eingegebenen Zusammensetzung und eine Messung der Eigenschaften der eingegebenen Zusammensetzung erfolgt, wonach eine Ausgabe der gemessenen Eigenschaften auf einem Nutzerinterface der Computersystems erfolgt und/oder die gemessenen Eigenschaften in der Datenbank gespeichert werden.

Die iterative Synthese und Prüfung (Bestimmung der Eigenschaften) der Versuchs-Zusammensetzungen zur Erweiterung des Trainingsdatensatzes kann vorteilhaft sein, da ein vollautomatisches oder - falls Nutzerbestätigung erforderlich ist - semiautomatisches System zur gezielten Erweiterung eines bestimmten, schon vorhandenen Trainingsdatensatzes zur iterativen Verbesserung eines neuronalen Netzwerks bereitgestellt wird. Das auf dem neuronalen Netzwerk basierte Vorhersageverfahren verbessert sich also iterativ durch entsprechende Steuerung der chemischen Anlage und automatische Verwendung der so erzeugten empirischen Daten zur Erweiterung des Trainingsdatensatzes.

Die Synthese und Prüfung (Bestimmung der Eigenschaften) der Prognosezusammensetzung kann vorteilhaft sein, da ein System bereitgestellt wird, in welches ein Nutzer nur die gewünschten Eigenschaften des chemischen Produkts spezifizieren muss, die Ermittlung der hierfür erforderlichen Komponenten und die Erzeugung des Produkts mit den gewünschten Eigenschaften laufen ab, sofern eine Prognosezusammensetzung vom neuronalen Netz für die im Eingangsvektor spezifizierten geforderten Eigenschaften ermittelt werden konnte.

Nach Ausführungsformen ist das Computersystem dazu ausgebildet, über Kommunikationsschnittstellen mit der Datenbank und/oder der Anlage zur Herstellung und Prüfung von thermoplastischen Zusammensetzungen für mechanisch und/oder thermisch belastete Bauteile zu kommunizieren. Kommunikationsschnittstellen sind dem Fachmann gekannt, z. B. SCSI, USB, FireWire, Bluetooth, Ethernet, WLAN, LAN, Bluetooth oder eine andere Netzwerkschnittstellen.

Nach Ausführungsformen beinhalten die Zusammensetzungen Compounds oder bestehen aus Compounds.

In einem weiteren Aspekt betrifft die Erfindung ein Computersystem zur Generierung thermoplastischer Zusammensetzungen für mechanisch und/oder thermisch belastete Bauteile. Das Computersystem umfasst eine Datenbank und ein Nutzerinterface und ist dazu konfiguriert, ein Verfahren zur Generierung einer Zusammensetzung gemäß Ausführungsformen der Erfindung durchzuführen.

In einem weiteren Aspekt betrifft die Erfindung ein Computerprogramm, ein digitales Speichermedium oder Computerprogrammprodukt mit von einem Prozessor ausführbaren Anweisungen, die bei Ausführung durch den Prozessor diesen dazu veranlassen, ein Verfahren zur Generierung einer Zusammensetzung gemäß Ausführungsformen der Erfindung durchzuführen.

In einem weiteren Aspekt betrifft die Erfindung ein System, welches das besagte Computersystem und eine Anlage umfasst. Die Anlage ist eine Anlage zur Herstellung und Prüfung von Zusammensetzungen für mechanisch und/oder thermisch belastete Bauteile. Die Anlage weist mindestens zwei Bearbeitungsstationen auf.

Unter einer "**Zusammensetzung**" wird hier eine Spezifikation eines chemischen Erzeugnisses verstanden, welche zumindest die Art der Rohstoffe ("Komponenten") spezifiziert, aus denen das chemische Erzeugnis gebildet wird. Wenn im Kontext dieser Anmeldung von der Herstellung oder Prüfung einer Zusammensetzung gesprochen wird, ist dies als Kurzfassung dafür zu verstehen, dass ein chemisches Erzeugnis gemäß den in der Zusammensetzung spezifizierten Angaben zu den Komponenten und optional auch deren Konzentrationen hergestellt wird bzw. dass dieses chemische Erzeugnis "geprüft", also dessen Eigenschaften messtechnisch erfasst, werden.

Unter einem **"Compound"** wird hier eine Zusammensetzung verstanden, welche neben der Angabe der Komponentenidentität, sowie der Mengen- oder Konzentrationsangaben der Komponenten auch die Herstellungsbedingungen umfasst.

Unter einer "**bekannten Zusammensetzungen**" wird eine Zusammensetzung verstanden, die ein chemisches Erzeugnis spezifiziert, dessen Eigenschaften zum Zeitpunkt des Trainings eines neuronalen Netzes der das Training durchführenden Person oder Organisation bekannt sind, da die bekannte Zusammensetzung schon einmal zur Herstellung eines chemischen Erzeugnisses verwendet und die Eigenschaften dieses Erzeugnisses empirisch gemessen wurden. Die Messung muss nicht notwendigerweise von dem Betreiber des chemischen Labors durchgeführt worden sein, welches nun die Prognosezusammensetzung ermittelt, sondern kann auch von anderen Labors durchgeführt und publiziert worden sein, sodass in diesem Fall die Eigenschaften der Fachliteratur entnommen sind. Da eine Zusammensetzung gemäß obiger Definition auch Compounds als Untermenge enthält, können die "bekannten Zusammensetzungen" gemäß Ausführungsformen der Erfindung auch "bekannte Compounds" enthalten oder "bekannte Compounds" sein.

Unter einer "**Versuchs-Zusammensetzung**" wird eine Zusammensetzung verstanden, die ein chemisches Erzeugnis spezifiziert, dessen Eigenschaften zum Zeitpunkt des Trainings eines neuronalen Netzes der das Training durchführenden Person oder Organisation nicht bekannt sind. Beispielsweise kann es sich bei einer Versuchs-Zusammensetzung um eine Zusammensetzung handeln, die manuell oder automatisch spezifiziert wurde, die aber noch nicht verwendet wurde, um ein entsprechendes chemisches Erzeugnis auch tatsächlich herzustellen. Entsprechend sind auch die Eigenschaften dieses Erzeugnisses nicht bekannt. Da eine Zusammensetzung gemäß obiger Definition auch Compounds als Untermenge enthält, können die "Versuchs-Zusammensetzungen" gemäß Ausführungsformen der Erfindung auch "Versuchs-Compounds" enthalten oder "Versuchs-Compounds" sein.

Unter einer "**Prognosezusammensetzung**" wird hier eine Zusammensetzung verstanden, für die ein trainiertes neuronales Netz vorhersagt (prognostiziert), dass diese ein chemisches Erzeugnis spezifiziert, dessen Eigenschaften einer von einem Benutzer vorgegebenen Spezifikation erwünschter Eigenschaften entsprechen. Beispielsweise kann die Spezifikation der erwünschten Eigenschaften dem neuronalen Netzwerk als Eingabe-Vektor bereitgestellt werden, der für jede der erwünschten Eigenschaften einen erwünschten oder akzeptablen Parameterwert oder Parameterwertbereich angibt.

Unter einer "**Datenbank**" wird hier jeglicher Datenspeicher oder Speicherbereich verstanden, in dem Daten, insbesondere strukturierte Daten, gespeichert sind. Bei der Datenbank kann es sich um eine oder mehre Textdateien, Spreadsheet-Dateien, ein Verzeichnis in einem Verzeichnisbaum, oder eine Datenbank eines relational oder objektorientiert strukturierten Datenbankmanagementsystem (DBMS), z. B. SQL wie MySQL oder PostgreSQL; XML oder noSQL wie N1 QL oder JSON handeln.

Unter einer "**Loss-Funktion**" (auch "Zielfunktion" genannt) eines Vorhersageproblems ist eine Funktion, die beim Training eines neuronalen Netzwerks verwendet wird und einen Wert ausgibt, dessen Betrag ein Hinweis auf die Qualität des prädiktiven Modells des trainierten neuronalen Netzwerk gibt und der im Zuge des Trainings minimiert werden soll, da der Betrag dieses Wertes die Fehlerhaftigkeit der Vorhersagen des neuronalen Netzes angibt.

Unter einer "**Anlage**" zur Herstellung und Prüfung von Zusammensetzungen wird hier ein aus mehreren Laborgeräten und optional einer Transporteinheit bestehendes System verstanden, welches in der Lage ist, die Laborgeräte und die Transporteinheit in orchestrierter Weise gemeinsam zu steuern um einen chemischen Workflow automatisch oder semi-automatisch durchzuführen. Bei dem Workflow kann es sich z. B. um einen Herstellungsworkflow oder einen Analyseworkflow oder eine Kombination aus beiden Workflows handeln.

Unter einer "**Prüfung von Zusammensetzungen**" durch die Anlage wird die messtechnische Erfassung ("Analyse") von Eigenschaften eines chemischen Erzeugnisses, das gemäß den Angaben in der Zusammensetzung erzeugt wurde, verstanden.

Unter einem "**Active Learning Modul**" wird ein Softwareprogramm oder ein Modul eines Softwareprogramms verstanden, welches dazu ausgebildet ist, gezielt eine (vergleichsweise kleine) Teil-Menge an Versuchs-Zusammensetzungen aus einer Menge von Versuchs-Zusammensetzungen so auszuwählen, dass nach Synthese und empirischer Messung der Eigenschaften dieser ausgewählten Versuchs-Zusammensetzung und der Berücksichtigung dieser Daten beim Training des neuronalen Netzwerks ein besonders starker Lerneffekt eintritt.

In den folgenden Abbildungen werden Ausführungsformen der Erfindung in exemplarischer Weise näher erläutert:
- Figur 1: zeigt ein Flussdiagramm eines Verfahrens zum Trainieren eines neuronalen Netzes und zur Verwendung des trainierten Netzes zur Vorhersage von Eigenschaften und/oder zur Vorhersage einer Zusammensetzung eines flüssigen Mediums;
- Figur 2: zeigt ein Blockdiagramm eines verteilten Systems zum Trainieren eines neuronalen Netzes und zur Verwendung des trainierten Netzes;
- Figur 3: zeigt einen 2D Ausschnitt eines mehrdimensionalen Datenraums, aus welchem das "Active Learning Modul" gezielt Datenpunkte auswählt;
- Figur 4: zeigt die Architektur eines neuronalen Netzes mit Eingabe- und Ausgabevektoren.

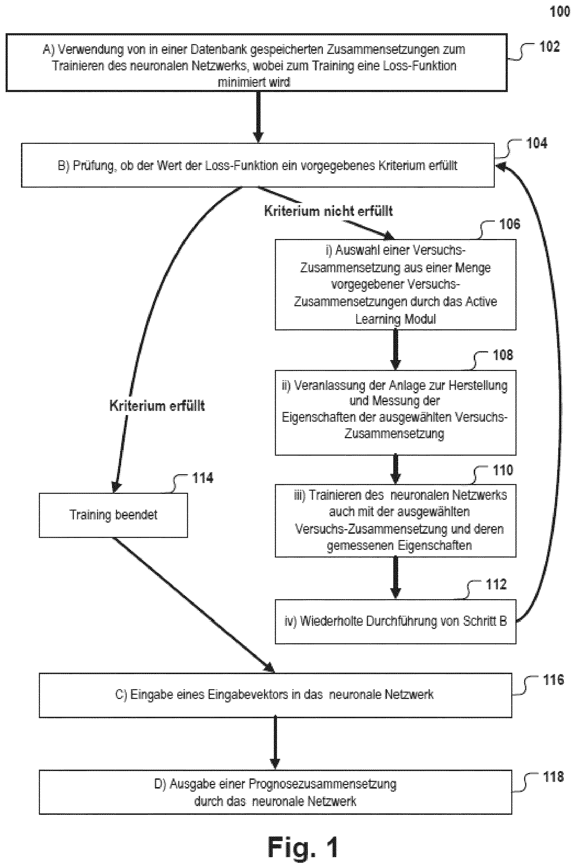

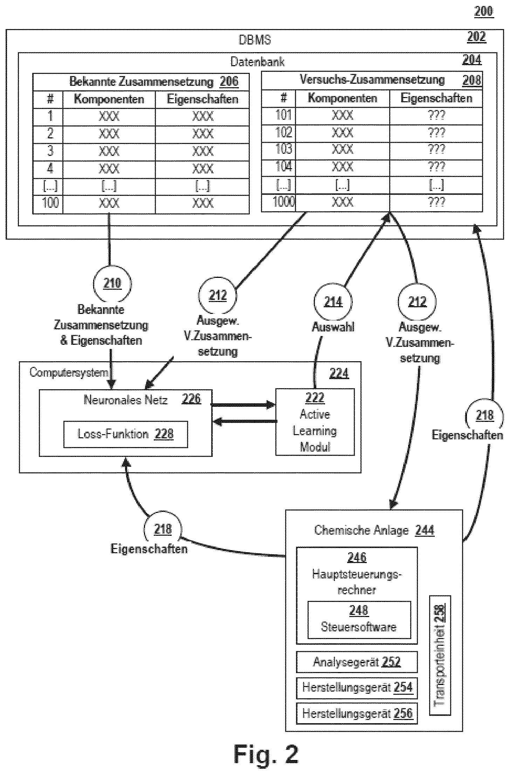

**Figur 1** zeigt ein Flussdiagramm eines computerimplementierten Verfahrens zur Generierung thermoplastischer Zusammensetzungen für mechanisch und/oder thermisch belastete Bauteile. Das Verfahren kann z. B. von einem Computersystem 224 wie in Figur 2 dargestellt ausgeführt werden.

In einem ersten Schritt 102 (a) werden bereits bekannte Zusammensetzungen als "initialer Trainingsdatensatz" verwendet um ein neuronales Netzwerk so zu trainieren, dass es in Antwort auf den Empfang eines Eingabevektors, der ein oder mehrere gewünschte Eigenschaften eines chemischen Erzeugnisses der oben genannten Kategorien enthält, eine PrognoseZusammensetzung vorhersagt, die diese gewünschten Eigenschaften aufweist. Die Prognosezusammensetzung spezifiziert zumindest die Art der Komponenten aus denen ein chemisches Erzeugnis der oben genannten Art (Bauteile) besteht und optional auch deren jeweilige Mengen bzw. Konzentrationen. Eine Kombination einer bekannten Zusammensetzung mit den bereits bekannten, empirisch ermittelten Eigenschaften des durch diese bekannte Zusammensetzung spezifizierten chemischen Erzeugnisses repräsentiert einen einzelnen Datenpunkt bzw. Datensatz innerhalb der Gesamtheit der initialen Trainingsdaten.

Im nächsten Schritt 104 (b) erfolgt eine Prüfung, ob der Wert einer Loss-Funktion ein vorgegebenes Kriterium erfüllt. Eine Erfüllung des Kriteriums drückt aus, dass die Vorhersagegenauigkeit des trainierten neuronalen Netzes als hinreichend anzusehen ist. Selektiv für den Fall, dass das Kriterium nicht erfüllt ist, werden die im Folgenden geschilderten Schritte 106-112 durchgeführt. Andernfalls wird das Training beendet (Schritt 114) und das fertig trainierte neuronale Netz zurückgegeben.

In Schritt 106 wählt das Active Learning Modul eine Versuchs-Zusammensetzung aus einer Menge vorgegebener Versuchs-Zusammensetzungen automatisch aus. Es gibt eine Vielzahl unterschiedlicher Active Learning Ansätze, die gemäß Ausführungsformen der Erfindung verwendet werden können.

Gemäß einer Implementierungsvariante folgt das Active Learning Modul dem "Expected model change" Ansatz und wählt diejenige Versuchs-Zusammensetzung aus, die (bei Neu-Trainieren des Netzwerks unter Berücksichtigung dieser Versuchs-Zusammensetzung und ihrer real gemessenen Eigenschaften) das derzeitige prädiktive Modell des trainierten neuronalen Netzwerks am stärksten verändern würde.

Gemäß einer anderen Implementierungsvariante folgt das Active Learning Modul dem "Expected error reduction" Ansatz und wählt diejenige Versuchs-Zusammensetzung aus, die einen Fehler des derzeitigen prädiktive Modell des trainierten neuronalen Netzwerks am stärksten reduzieren würde.

Gemäß einer weiteren Implementierungsvariante folgt das Active Learning Modul dem "Minimum Marginal Hyperplane" Ansatz und wählt diejenige Versuchs-Zusammensetzung aus, die einer Trennlinie bzw. Trennebene, die in einem mehrdimensionalen Datenraum vom derzeitigen prädiktiven Modell des trainierten neuronalen Netzwerks aufgespannt wird, am nächsten liegt. Bei der Trennlinie bzw. Trennebene handelt es sich um Grenzflächen innerhalb des mehrdimensionalen Datenraums, in welchem das prädiktive Modell eine Klassifikationsentscheidung trifft, also Datenpunkte auf der einen Seite der Trennlinie bzw. Trennebene einer anderen Klasse bzw. Kategorie zuordnet als den Datenpunkten auf der anderen Seite der Trennlinie. Diese Nähe der Datenpunkte zu der Trennebene wird so interpretiert, dass das prädiktive Modell sich bezüglich einer Klassifikationsentscheidung unsicher ist und in besonders starkem Maße davon profitieren würde, wenn real gemessene Datensätze (bestehend aus einer Kombination aus Komponenten und optional deren Konzentrationen und den gemessenen Eigenschafen des gemäß dieser Zusammensetzung der Komponenten erzeugten chemischen Erzeugnisses) aus der Nähe dieser Trennebene zusätzlich gemessen werden, um damit das neuronale Netz weiter zu trainieren.

Nach der Auswahl von einer der Versuchs-Zusammensetzungen aus der Datenbank veranlasst in Schritt 108 das Computersystem eine Anlage zur Herstellung und Prüfung von chemischen Zusammensetzungen so, dass ein chemisches Erzeugnis gemäß den Angaben in der ausgewählten Versuchs-Zusammensetzung ggf. automatisch hergestellt und geprüft wird. Unter dieser Prüfung wird ein messtechnisches Erfassen von ein oder mehreren Eigenschaften des chemischen Erzeugnisses verstanden, also z. B. das Messen von Aminoendgruppengehalt, Carboxylendgruppengehalt, , Durchgangwiderstand, Schmelze-Volumenfließrate (MVR), Glasübergangstemperatur, Brennbarkeit (UL94), Kerbschlagzähigkeit, Bruchspannung, Bruchdehnung, Streckspannung E-Modul, Wärmebeständigkeitoder dergleichen.

Die in Schritt 108 erhaltenen real gemessenen Eigenschaften werden verwendet, um die ausgewählte Versuchs-Zusammensetzung zu ergänzen, sodass ein vollständiger weiterer Datenpunkt bestehend aus einer bekannten Zusammensetzung und bekannten Eigenschaften entsteht, welcher zur Erweiterung des in a) bzw. vorigen Iterationen verwendeten Trainingsdatensatzes dient. In Schritt 110 wird das neuronale Netzwerk also auf einem erweiterten Trainingsdatensatz neu trainiert. Dies kann je nach Implementierungsvariante so erfolgen, dass das Training noch einmal komplett auf Basis des erweiterten Trainingsdatensatzes vorgenommen wird, oder das Training in Schritt 110 erfolgt inkrementell, sodass das bisher Gelernte beibehalten und durch die Berücksichtigung des neuen Trainings-Datenpunkts nur modifiziert wird.

In Schritt 112 wird eine wiederholte Prüfung der Vorhersagequalität des trainierten neuronalen Netzes angestoßen und die Schritte 104-112 so lange wiederholt, bis das Netzwerk eine hinreichende Vorhersagequalität besitzt, was daran erkennbar ist, dass die Loss-Funktion das Kriterium erfüllt, also z. B. der von der Loss-Funktion berechnete "Fehlerwert" unterhalb eines vordefinierten Maximalwerts ist.

Das fertig trainierte neuronale Netz kann nun dazu verwendet werden, sehr schnell und zuverlässig Zusammensetzungen vorherzusagen, welche eine oder mehrere gewünschte Eigenschaften aufweisen (eine sogenannte "Prognosezusammensetzung"). Hierfür gibt ein Nutzer in Schritt 116 einen Eingangsvektor in das trainierte neuronale Netz ein, welcher ein oder mehrere der gewünschten Eigenschaften spezifiziert. Beispielsweise können die Elemente des Eingangsvektors aus einem numerischen Wert oder Wertebereich bestehen, der als erwünschter bzw. akzeptabler Wert bzw. Wertebereich zu verstehen ist. Beispielsweise kann es ein Erfordernis sein, eine Zusammensetzung mit einer Leitfähigkeit in einem bestimmten Wertebereich und einer Farbe in einem bestimmten Farbbereich herzustellen.

Da das Netz in mehreren Iterationen basierend auf einem sinnvoll und gezielt erweiterten Trainingsdatensatz die statistischen Korrelationen zwischen Komponenten (und optional auch deren Konzentrationen) und den Eigenschaften des resultierenden chemischen Erzeugnisses gelernt hat, kann das trainierte neuronale Netz nun in Schritt 118 eine Prognosezusammensetzung vorhersagen, die die gewünschten Eigenschaften aufweist, und diese an den Nutzer und/oder an eine chemische Anlage zur unmittelbaren Synthese ausgeben.

Ziel des Trainings des neuronalen Netzes ist es, dass das trainierte Netz anhand einer eingegebenen Menge an gewünschten Eigenschaften und entsprechender Parameterwertbereiche eine Prognosezusammensetzung vorhersagen kann, also einer Spezifikation zumindest der Art und Anzahl und optional auch die jeweiligen Mengen der Komponenten eines chemischen Erzeugnisses, das die gewünschten Eigenschaften aufweist. Wendet man die Vorhersage auf eine Test-Zusammensetzung an, deren Komponenten bekannt und deren tatsächliche Eigenschaften empirisch gemessen wurden, gibt es also entweder ein richtiges oder falsches Vorhersageergebnis. Zur Beurteilung der Vorhersagequalität eines trainierten neuronalen Netzes wird wie oben erwähnt die "Loss-Funktion" verwendet.

Eine "Loss-Funktion" (auch "Zielfunktion" genannt) für ein Vorhersageproblem, das auch als Klassifikationsproblem verstanden werden kann, kann z. B. im einfachsten Fall nur die korrekt erkannten Vorhersagen aus einer Menge an Vorhersagen zählen. Je höher der Anteil der korrekten Vorhersagen, desto höher ist die Qualität des prädiktiven Modells des trainierten neuronalen Netzwerk. Beispielsweise kann die Frage, ob eine elektrische Eigenschaft wie die Leitfähigkeit innerhalb eines vordefinierten akzeptablen Bereiches liegt, als Klassifikationsproblem verstanden werden.

Es sind aber auch viele alternative Loss-Funktionen und entsprechende Kriterien zur Beurteilung der Vorhersagegenauigkeit des trainierten neuronalen Netzes möglich. Beispielsweise kann das Netzwerk erwünschte Eigenschaften in Form eines Eingabevektors empfangen und diese Daten verwenden, um die Art der Komponenten der Zusammensetzung vorherzusagen. In diesen Anwendungsfällen soll das neuronale Netzwerk einen Wert schätzen und keine Klasse, also z. B. den die Menge einer Komponente einer Zusammensetzung. Diese Anwendungsfälle nennt man Regressionsprobleme. Dafür ist eine andere Zielfunktion notwendig. Beispielsweise kann eine Loss-Funktion für Regressionsprobleme in einer Funktion bestehen, die berechnet, wie stark sich der vom Netzwerk vorhergesagte Wert von dem tatsächlich gemessenen Wert unterscheidet. Beispielsweise kann die Loss-Funktion eine Funktion sein, deren Ausgabewert positiv korreliert mit dem aggregierten Wert der Abweichung jeder vorhergesagten Komponente von einer tatsächlich verwendeten Komponente einer hergestellten Zusammensetzung. Der aggregierte Wert kann z. B. ein arithmetisches Mittel sein. Beispielsweise kann die Qualität eines trainierten neuronalen Netzes dann als hinreichend angesehen werden, wenn diese aggregierten Abweichungen (Fehler) aller durch das Netzwerk vorhergesagten Komponenten einer Zusammensetzung bezüglich der tatsächlich verwendeten Komponenten unterhalb eines vordefinierten Schwellenwertes liegt. In diesem Fall kann das neuronale Netz zur Vorhersage von unbekannten Zusammensetzungen verwendet werden, die ein chemisches Erzeugnis spezifizieren, die eine oder mehrere gewünschte Eigenschaften aufweist. Wenn diese aggregierten Abweichungen der vorhergesagten Komponenten von den tatsächlich verwendeten Komponenten dagegen stärker als in dem vorgegebenen Maximalwert abweichen, wird automatisch das Active Learning Modul dazu veranlasst, den Trainingsdatensatz zu erweitern, indem es eine weitere Versuchs-Zusammensetzung auswählt, deren Synthese und Eigenschaftsanalyse durch die chemische Anlage veranlasst und die ausgewählte Versuchs-Zusammensetzung zusammen mit den für diese gemessenen Eigenschaften als zusätzlichen Datenpunkt in einem erweiterten Trainingsdatensatz zum erneuten Trainieren des neuronalen Netzes auf dem erweiterten Trainingsdatensatz verwendet.

Der Eingabevektor, der bei jedem iterativen Neu-Trainieren auf Basis der durch die Anlage ermittelten Eigenschaften gebildet wird, beinhaltet die empirisch gemessen Eigenschaften eines gemäß des ausgewählten Versuchs-Zusammensetzung hergestellten Produkts. Der Ausgabevektor beinhaltet eine Menge an vorhergesagten Komponenten der ausgewählten Versuchs-Zusammensetzung, sodass mit der Loss-Funktion die vorhergesagten Komponenten mit den tatsächlichen Komponenten der Versuchs-Zusammensetzung verglichen werden können. Der verwendete Eingabevektor zum Testen der Loss-Funktion ist vorzugsweise bei jeder Iteration der gleiche, sodass Veränderungen des von der Loss-Funktion berechneten Fehlerwertes auf Änderungen im prädiktiven Modell des Netzwerks und nicht auf Änderungen im Eingabevektor zurückgeführt werden können. In manchen Ausführungsformen wird die Loss-Funktion auch für mehrere Test-Zusammensetzungen mit empirisch bekannten Eigenschaften angewendet, um die Datenbasis bei der Bestimmung, ob die Loss-Funktion ein bestimmtes Kriterium erfüllt, zu verbreitern.

Nachdem das Training des Netzes abgeschlossen ist, kann das trainierte neuronale Netz zur Vorhersage einer Prognosezusammensetzung auf Basis eines Eingabevektors, welcher mehrere gewünschte Eigenschaften und entsprechende Parameterwertbereiche spezifiziert, erzeugen und ausgeben. Die Ausgabe kann an einen Nutzer erfolgen und/oder an die Anlage und die Anlage automatisch dazu zu veranlassen, ein chemisches Erzeugnis gemäß der Prognosezusammensetzung herzustellen und empirisch zu prüfen, ob dieses die gewünschten Eigenschafen aufweist.

Ganz allgemein kann das beschriebene Verfahren insbesondere für die Berechnung von Prognosezusammensetzung im Kontext der Herstellung von mechanisch und/oder thermisch belasteten Bauteilen vorteilhaft sein, da eine Vorhersage einer geeigneten Zusammensetzung aufgrund der Vielzahl an Komponenten und deren Wechselwirkungen kaum möglich ist.

Beispielsweise können im Falle von elektrisch leitfähigen Thermoplasten unterschiedliche Hilfsmittel zugesetzt werden, z. B. leitfähige Partikel wie Ruß, Carbo-Nano-Tubes (CNT), Graphen usw., oder Salze, z. B. ionische Flüssigkeiten, Komplexsalze usw.. Derartige Zusammensetzungen werden dann über ihre Leitfähigkeiten oder Widerstände, z. B. Durchgangswiderstand, Oberflächenwiderstand, charakterisiert. Die an sich Isolatoren (Materialien mit sehr hohem elektrischen Widerstand) darstellenden Thermoplasten werden durch Zugabe unterschiedlicher Klassen von Substanzen also begrenzt leitfähig ausgestattet und die mindestens zwei Zusätze bewirken beide ein Zunahme der Leitfähigkeit. Weiterhin beeinflussen diese Zusätze natürlich auch andere Eigenschaften, wie z. B. die Viskosität, oder sie sind sehr unterschiedlich teuer. Außerdem können die Zusätze synergistisch wirksam sein.

**Figur 2** zeigt ein Blockdiagramm eines verteilten Systems 200 zum Trainieren eines neuronalen Netzes 226 und zur Verwendung des trainierten Netzes zur Vorhersage von Zusammensetzungen, insbesondere von thermoplastischen Zusammensetzungen für mechanisch und/oder thermisch belastete Bauteile.

Das System beinhaltet eine Datenbank 204 mit bekannten Zusammensetzungen 206 sowie mit Versuchs-Zusammensetzungen 208. Wie bereits vorab beschrieben, kann die Datenbank im einfachsten Fall aus einem Speicherbereich bestehen, in dem ein oder mehrere Dateien, zum Beispiel Textdateien oder kommaseparierten Dateien, abgespeichert sind. Bei der in Figur 2 dargestellten Ausführungsform ist die Datenbank 200 die Datenbank eines Datenbankmanagementsystem (DBMS), zum Beispiel eines relationalen DBMS wie zum Beispiel MySQL oder objektorientiert DBMS wie zum Beispiel JSON. Insbesondere bei größeren Datensätzen ist die Verwendung eines relationalen oder objektorientierten DBMS zur Verwaltung und schnellen Abfrage der Datensätze vorteilhaft, dass sich Abfragen genauer spezifizieren und schneller ausführen lassen. Beispielsweise können die bekannten Zusammensetzungen 206 in einer ersten Datenbanktabelle und die Versuchs-Zusammensetzungen 208 in einer weiteren Datenbanktabelle gespeichert sein. Es ist jedoch auch möglich, sämtliche bekannte Zusammensetzungen und Versuchs-Zusammensetzungen in einer einzigen Tabelle zu speichern und die unterschiedlichen Typen an Datensätzen durch Metadaten/"Flags" entsprechend zu kennzeichnen. Die Art der Datenablage kann vom Fachmann frei gewählt werden, solange die Art der Speicherung eine logische Unterscheidung der beiden Zusammensetzungstypen ermöglicht.

Bei den bekannten Zusammensetzungen 206 kann es sich beispielsweise um eine Menge von Datensätzen handeln, die jeweils eine Zusammensetzung, welche bereits mindestens einmal tatsächlich verwendet wurde, um ein entsprechendes chemisches Erzeugnis herzustellen, und die physikalischen, chemischen, haptischen, optischen und/oder sonstige messtechnisch erfassbaren Eigenschaften dieses Erzeugnisses beinhaltet. Beispielsweise kann es sich bei den bekannten Zusammensetzungen 206 um die Gesamtheit derjenigen Zusammensetzungen handeln, die von einer bestimmten Organisation, oder von einem bestimmten Labor, oder von einer bestimmten Laboranlage 244 bisher bereits hergestellt wurden und von welchen auch zumindest einige der oben genannten messtechnisch erfassbaren Parameter (Eigenschaften) empirisch erfasst wurden.

Die in der Datenbank 204 gespeicherten bekannten Zusammensetzungen 206 zeichnen sich also dadurch aus, dass nicht nur ihre Komponenten (also die einzelnen chemischen Bestandteile und derer jeweiligen Mengen bzw. Konzentrationsangaben), sondern auch zumindest einige messtechnisch erfassbare Eigenschaften des chemischen Erzeugnisses, welches gemäß dieser Zusammensetzung hergestellt wurde, bekannt sind. Jede bekannte Zusammensetzung eines chemischen Erzeugnisses ist also in der Datenbank 204 als Datensatz repräsentiert, welcher die Komponenten dieses Erzeugnisses sowie die besagten messtechnisch erfassten Eigenschaften dieses Erzeugnisses beinhaltet.

Die in der Datenbank 204 gespeicherten Versuchs-Zusammensetzungen 208 sind dagegen Zusammensetzungen, deren physikalische, chemische, optische und/oder sonstige messtechnisch erfassbare Eigenschaften zumindest dem Betreiber der Datenbank und/oder der Laboranlage 244 nicht bekannt sind. Dies ist in Figur 2 durch Fragezeichen angedeutet. Eine Versuchs-Zusammensetzung ist also in der Datenbank 204 als Datensatz repräsentiert, der zwar die Komponenten (also die einzelnen chemischen Bestandteile und optional auch deren jeweiligen Mengen bzw. Konzentrationsangaben) eines chemischen Erzeugnisses charakterisieren, nicht jedoch die besagten messtechnisch erfassbaren Eigenschaften dieses Erzeugnisses. Beispielsweise können die besagten Eigenschaften deshalb nicht vorhanden sein, weil die entsprechende Zusammensetzung noch niemals oder zumindest nicht von dem entsprechenden Labor bzw. der Laboranlage verwendet wurde, um ein entsprechendes chemisches Erzeugnis zu synthetisieren.

In manchen Ausführungsformen können die Versuchs-Zusammensetzungen 208 von einem Fachmann manuell erstellt und in der Datenbank gespeichert werden. Beispielsweise kann ein Chemiker basierend auf seinen Erfahrungen bei der Herstellung von mechanisch und/oder thermisch belasteten Bauteilen und deren jeweiligen Eigenschaften neue Versuchs-Zusammensetzungen spezifizieren, von welchen der Fachmann erwartet, dass diese bestimmte gewünschte Materialeigenschaften aufweisen. Beispielsweise können die Versuchs-Zusammensetzungen dadurch generiert werden, dass der Fachmann eine bekannte Zusammensetzung abändert, indem er einzelne Komponenten weglässt oder neu hinzufügt. Falls die Zusammensetzung auch Konzentrationen der ein oder mehreren Komponenten, sowie die Herstellungsparameter umfasst ("Compound"), kann eine solche erweiterte Versuchs-Zusammensetzung auch dadurch gebildet werden, dass die Konzentrationen der Komponenten in bekannten Zusammensetzungen geändert werden.

In anderen Ausführungsformen werden die Versuchs-Zusammensetzungen 208 automatisch erstellt und in der Datenbank 204 gespeichert. Beispielsweise kann jede der bekannten Zusammensetzungen 206 aus 20 unterschiedlichen chemischen Komponenten bestehen. Die Versuchs-Zusammensetzungen 208 werden nun automatisch dadurch erzeugt, dass einzelne Komponenten der Zusammensetzung durch andere Substanzen ersetzt werden.

Falls es sich bei den Zusammensetzungen um "erweiterte" Zusammensetzungen mit Konzentrationsangaben handelt, können Versuchs-Zusammensetzungen auch dadurch gebildet werden, dass die Mengen einzelner Komponenten der bekannten Zusammensetzungen 206 variiert werden, also zum Beispiel um 10 % erhöht und/oder um 10 % erniedrigt werden. Wird jeweils nur immer eine einzige Komponente variiert, in dem eine um 10 % erhöhte sowie um eine um 10 % erniedrigte Menge dieser Komponente verwendet wird, werden pro Komponente also zwei Varianten gebildet. Bei 20 Komponenten entstehen mit diesem Verfahren also 40 Versuchs-Zusammensetzungen. Die Zahl der automatisch generierten Versuchs-Zusammensetzungen wird vorzugsweise noch weiter dadurch erhöht, dass zwei oder mehr Komponenten gleichzeitig in ihrer Konzentration gegenüber ihrer Konzentration in der bekannten Zusammensetzung und 10 % erhöht oder erniedrigt werden. Rein kombinatorisch können auf diese Weise 2²⁰ = 1048576 erweiterte Versuchs-Zusammensetzungen automatisch erzeugt werden. Die Anzahl der Versuchs-Zusammensetzungen kann weiterhin drastisch dadurch erhöht werden, dass noch mehr Konzentrationsvarianten, also zum Beispiel -20 %, -10 %, +10 %, +20 % für jede der 20 Komponenten verwendet werden und/oder dadurch, dass chemische Komponenten weggelassen oder zusätzlich verwendet werden. Die Menge der Versuchs-Zusammensetzungen kann daher, insbesondere bei einer automatischen Erzeugung der Versuchs-Zusammensetzungen, sehr hoch sein.

In manchen Ausführungsformen umfassen die Versuchs-Zusammensetzungen 208 sowohl manuell erstellte Versuchs-Zusammensetzungen als auch automatisch generierte Versuchs-Zusammensetzungen.

Beispielsweise kann eine automatische Erzeugung der Versuchs-Zusammensetzungen vorteilhaft sein, da dadurch auf schnelle Weise ein sehr großer Parameterraum aus Komponenten und optional auch deren Konzentrationen abgedeckt werden kann, der typischerweise, wenn ein sprechender Versuchs-Zusammensetzungen-Erzeugungsalgorithmus verwendet wird, die einzelnen Komponenten und deren Konzentrationen breitflächig und grobmaschig abdeckt.

Die manuell ergänzten Versuchs-Zusammensetzungen können Versuchs-Zusammensetzungen sein, von welchen sich ein Fachmann auf Basis seines Erfahrungswissens einen besonders hohen Lerneffekt für das neuronale Netz verspricht oder von deren Synthese sich der Fachmann aus sonstigen Gründen Erkenntnisvorteile verspricht.

In Figur 2 wird die in der Regel große Anzahl an Versuchs-Zusammensetzungen dadurch angedeutet, dass die Datenbank 204 nur 100 bekannte Zusammensetzungen aber 900 Versuchs-Zusammensetzungen umfasst. Das tatsächliche Zahlenverhältnis von bekannten Zusammensetzungen und Versuchs-Zusammensetzungen hängt jedoch stark vom Einzelfall ab, also zum Beispiel davon, wie viele Zusammensetzungen von einem bestimmten Labor bereits erzeugt und deren chemische Eigenschaften bestimmt wurden, ob die Datenbank bekannte Zusammensetzungen und deren Eigenschaften aus externen Quellen integriert hat, und/oder ob die Versuchs-Zusammensetzungen manuell oder automatisch erstellt wurden. Es ist also durchaus möglich, dass die Datenbank 204 mehrere 1000 bekannte Zusammensetzungen beinhaltet. Typischerweise ist die Menge der Versuchs-Zusammensetzungen erheblich größer als die Menge an empirischen Syntheseversuchen, die ein Labor im Hinblick auf Kosten und Rentabilität tatsächlich physikalisch durchführen kann.

Das verteilte System 200 beinhaltet ferner ein Computersystem 224, welches ein neuronales Netz 226 und ein Aktive Learning Modul 222 beinhaltet. Das Aktive Learning Modul 222 hat Zugriff auf die Datenbank 204. Bei dem Zugriff handelt es sich zumindest um einen Lesezugriff, um ein oder mehrere ausgewählte Versuchs-Zusammensetzungen und deren Komponenten aus der Datenbank 204 auslesen zu können. Nach manchen Ausführungsformen hat das Aktive Learning Modul und/oder eine chemische Anlage 244, die ein chemisches Erzeugnis gemäß der ausgewählten Versuchs-Zusammensetzungen hergestellt und messtechnisch analysiert, auch Schreibrechte auf die Datenbank 204, um die für die ausgewählte Versuchs-Zusammensetzung messtechnisch erfassten Eigenschaften in der Datenbank zu speichern. Beispielsweise kann die Speicherung der messtechnisch erfassten Eigenschaften einer ausgewählten und neu synthetisieren Versuchs-Zusammensetzung dazu führen, dass diese Versuchs-Zusammensetzung zu einer bekannten Zusammensetzung wird und entsprechend in der Datenbank 204 an einem anderen Ort gespeichert und/oder mit anderen Metadaten ("Flags") versehen wird. Beispielsweise kann das DBMS 202 auf einem Datenbankserver installiert sein, sodass der Zugriff des Aktive Learning Moduls und/oder der chemischen Anlage 244 auf die Datenbank 204 über ein Netzwerk erfolgt. Bei dem Netzwerk kann es sich insbesondere um das Intranet einer Organisation, aber auch um das Internet handeln.

Auch andere Systemarchitekturen sind möglich. So kann beispielsweise die Datenbank 204 bzw. das DBMS 202 auch Bestandteil des Computersystems 224 oder des Hauptsteuerungsrechners 246 sein und/oder das neuronale Netz 226 und das Active Learning Modul 222 können auf unterschiedlichen Computersystemen installiert sein. Unabhängig von der tatsächlich gewählten Architektur muss ein Austausch der Daten 210, 212, 214, 218 wie z. B. in Figur 2 dargestellt möglich sein derart, dass alle am Verfahren teilnehmenden Komponenten die benötigten Inputdaten von anderen Komponenten erhalten können. Der Datenaustausch kann dabei direkt oder indirekt über weitere Komponenten wie zum Beispiel Gateways erfolgen. Beispielsweise kann in manchen Ausführungsformen die chemische Anlage die für die ausgewählte Versuchs-Zusammensetzung messtechnisch erfassten Eigenschaften direkt in die Datenbank 204 speichern oder nur an das Computersystem 224 senden, welches die Eigenschaften dann so in der Datenbank 204 speichert, dass der Datensatz der Versuchs-Zusammensetzung um die Eigenschaften ergänzt wird und dadurch zu einer "bekannten Zusammensetzung" wird.

Gemäß einer weiteren alternativen Systemarchitektur handelt es sich bei dem Computersystem 224 und dem Hauptsteuerungsrechner 246 um das gleiche Computersystem.

Gemäß Ausführungsformen der Erfindung ist das Computersystem 224 bzw. sind die auf diesem installierten Komponenten 226, 222 dazu ausgebildet, zunächst die bekannten Zusammensetzungen 206 aus der Datenbank auszulesen und die bekannten Zusammensetzungen 206 als Trainingsdatensatz 210 zum initialen Trainieren des neuronalen Netzes 226 zu verwenden. Im Zuge des Trainings des neuronalen Netzes wird ein prädiktives Modell des neuronalen Netzes erzeugt, welches basierend auf dem Trainingsdatensatz die Beziehungen zwischen den zur Synthese eines chemischen Erzeugnisses verwendeten Komponenten (also zwischen einer Zusammensetzung) und den messtechnisch erfassten Eigenschaften des hergestellt Erzeugnisses abbildet. Mit Hilfe dieses im Zuge des Trainings gewonnenen prädiktiven Modells kann das trainierte neuronale Netz auf Basis einer in Form eines Eingabevektors eingegebenen gewünschten Eigenschaften die Zusammensetzung vorhersagen, welche ein Erzeugnis spezifiziert, das voraussichtlichen die erwünschten Eigenschaften aufweist. Beispielsweise kann es eine erwünschte Eigenschaft sein, dass die Leitfähigkeit des chemischen Erzeugnisses innerhalb eines bestimmten Parameterwertbereiches liegt.

Aufgrund der begrenzten Zahl der bekannten Zusammensetzungen 206 und ihrer Eigenschaften ist das nach der initialen Trainingsphase erhaltene trainierte neuronale Netz 226 in manchen Fällen noch nicht in der Lage, die Komponenten einer Zusammensetzung basierend auf einer Liste gewünschter Eigenschaften mit hinreichender Verlässlichkeit vorherzusagen. Hierzu ist die verwendete Datenbasis oftmals zu klein.

Eine Erweiterung des Trainingsdatensatz dadurch, dass sämtliche Versuchs-Zusammensetzungen in der Anlage 244 hergestellt und danach deren Eigenschaften messtechnisch bestimmt werden, ist zumeist zu teuer und/oder zu aufwändig. Gemäß Ausführungsformen der Erfindung ermöglicht es die Verwendung des Active Learning Moduls, gezielt einige wenige Versuchs-Zusammensetzungen auszuwählen und nur für diese in der chemischen Anlage 244 ein entsprechendes chemisches Erzeugnis synthetisieren und analysieren zu lassen, um mit möglichst wenigen Synthesen (und damit mit möglichst hoher Effizienz) den Trainingsdatensatz so zu erweitern, dass die Vorhersagekraft des prädiktiven Modells des neuronalen Netzes 226 durch ein erneutes Trainieren auf dem erweiterten Trainingsdatensatz deutlich zu verbessern. Nach dem erneuten Trainieren des neuronalen Netzes auf dem erweiterten Datensatz wird die Vorhersagekraft des neuronalen Netzes erneut unter Verwendung der Loss-Funktion 228 geprüft. Wird dabei ein vorgegebenes Kriterium nicht erfüllt, überschreitet also zum Beispiel der Loss-Wert einen vorgegebenen Maximalwert, bedeutet das, dass die Qualität des neuronalen Netzes bzw. dessen prädiktiven Modells immer noch nicht hoch genug ist und ein erneutes Training auf einem noch stärker erweiterten Trainingsdatensatz stattfinden soll. In diesem Fall führt das Active Learning Modul einen erneuten Auswahlschritt bezüglich einer Versuchs-Zusammensetzung durch, die bisher noch nicht ausgewählt und für eine Synthese des entsprechenden Mediums verwendet wurde. Es wird wie beschrieben eine Synthese eines chemischen Erzeugnisses basierend auf der ausgewählten Versuchs-Zusammensetzung und eine messtechnische Erfassung der Eigenschaften dieses Erzeugnisses vorgenommen, sodass die ausgewählte Versuchs-Zusammensetzung zusammen mit den gemessenen Eigenschaften als neuer Trainings-Datensatz den bestehenden Trainings-Daten hinzugefügt werden kann, um das neuronale Netzwerk 226 basierend auf einem noch stärker erweiterten Trainingsdatensatz neu zu trainieren. In mehreren Iterationen können somit durch automatische und gezielte Auswahl von Versuchs-Zusammensetzungen, die eine besonders starke Verbesserung des prädiktiven Modells des neuronalen Netzes versprechen, sowie automatisch ausgeführte Synthese- und Analyseschritte auf Basis der jeweils ausgewählten Versuchs-Zusammensetzung, die Vorhersagekraft des neuronalen Netzes auf effiziente Weise schnell verbessert werden. Wenn nach einer Iteration festgestellt wird, dass die Loss-Funktion das Kriterium erfüllt, ist eine Erweiterung des Trainingsdatensatzes nicht mehr nötig, da die Vorhersagekraft des trainierten neuronalen Netzwerks als ausreichend angesehen werden kann.

Die Identifikation der auszuwählenden Versuchs-Zusammensetzung kann z. B. so erfolgen wie in Figur 2 dargestellt. Das Active Learning Modul 222 ist dazu konfiguriert, die identifizierte Versuchs-Zusammensetzung aus der Datenbank 204 auszulesen (z. B. mittels eines SELECT Befehls 214 an eine relationale Datenbank) und an die chemische Anlage 244 zu übermitteln. Das Computersystem 224 beinhaltet also gegebenenfalls eine Schnittstelle zur Kommunikation mit der Anlage 244 und/oder ist Bestandteil der Anlage. Die Anlage ist dazu konfiguriert, ein chemisches Erzeugnis auf Basis der ausgewählten Versuchs-Zusammensetzung 212 zu synthetisieren und ein oder mehrere Eigenschaften des hergestellten Erzeugnisses zu messen. Beispielsweise kann die Anlage mehrere Synthesegeräte 254, 256 oder Synthesemodule und mehrere Analysegeräte 252 (oder Analysemodule) beinhalten, die jeweils ein oder mehrere Schritte bei der Synthese oder Analyse chemischer Erzeugnisse oder deren Zwischenstufen durchführen. Die Anlage verfügt ferner gegebenenfalls über eine oder mehrere Transporteinheiten 258, z. B. Laufbänder oder Roboterarme, die die Komponenten, Zwischenprodukte und Verbrauchsmaterialien zwischen den verschiedenen Synthese- und/oder Analyseeinheiten hin- und her transportieren. Die Anlage 244 umfasst einen Hauptsteuerungsrechner 246 mit einer Steuersoftware 248 oder ist an einen solchen Rechner 248 operativ über ein Netzwerk gekoppelt. Die Steuersoftware 248 ist dazu konfiguriert, die von den Synthese- und Analyseeinheiten und optional der Transporteinheit durchgeführten Synthese- Analyse- und/oder Transportschritte so zu koordinieren, dass ein chemisches Erzeugnis gemäß den Angaben in der ausgewählten Zusammensetzung 212 hergestellt und dessen Eigenschaften messtechnisch erfasst werden. Vorzugsweise speichert die Steuerungssoftware die erfassten Eigenschaften 218 der neu hergestellten ausgewählten Zusammensetzung (direkt oder über die Vermittlung des Computers 224) in der Datenbank 204 so, dass die Eigenschaften mit der ausgewählten Versuchs-Zusammensetzung verknüpft gespeichert sind. Hierbei wird also der "unvollständige" Datensatz der ausgewählten Versuchs-Zusammensetzung um die messtechnisch in der Anlage 244 erfassten Eigenschaften ergänzt und dadurch in eine "bekannte Zusammensetzung" umgewandelt.

Die Eigenschaften 218 der ausgewählten Versuchs-Zusammensetzung werden zudem an das Computersystem 224 übermittelt, sodass eine Kombination der vom Active Learning Modul ausgewählten Zusammensetzung 212 und deren Eigenschaften 218 einen neuen vollständigen Datensatz ergibt, um welchen die Gesamtheit der Trainingsdaten erweitert wird. Das neuronale Netz wird auf Basis des erweiterten Trainingsdatensatzes neu trainiert und der Effekt der Erweiterung der Trainingsdaten auf die Qualität der Vorhersagen des neuronalen Netzes mittels der Loss-Funktion 228 getestet. Falls der Wert der Loss-Funktion ein vordefiniertes Kriterium erfüllt, also z. B. nur einen Loss-Wert unterhalb eines Maximalwerts hat, kann das Training beendet werden. Anderenfalls wird das Ergebnis der Kriteriumsprüfung an das Active Learning Modul übermittelt und dieses dazu veranlasst, eine weitere Versuchs-Zusammensetzung auszuwählen.

Gemäß manchen Ausführungsformen ist auch die Anlage 244 oder auch mehrere Anlagen zur Synthese und Analyse chemischer Erzeugnisse Bestandteil des verteilten Systems 200.

Bei der Anlage 244 kann es sich um eine Hochdurchsatzanlage handeln, z. B. um eine Hochdurchsatzanlage zur Herstellung und Analyse von thermoplastischen Polymeren und Bauteilen. Beispielsweise kann die Anlage ein System zum automatischen Testen und zur automatischen Herstellung chemischer Produkte sein wie es in der WO 2017/072351 A2 beschrieben ist.

Der Fachmann kann mit Hilfe des in Figur 2 dargestellten Systems also vermeiden, dass auf ungezielte und aufwändige Weise eine Vielzahl von Iterationsstufen und Komponentenzusammensetzungen hergestellt und analysiert werden müssen, um einen hinreichend großen Trainingsdatensatz zu erhalten. Aufgrund der enormen Komplexität der Abhängigkeiten von Komponenten, ihren jeweiligen Kombinationen und Konzentrationen voneinander wie auch der Komplexität der Abhängigkeiten der verschiedenen messtechnisch erfassbaren Eigenschaften chemischer Erzeugnisse von ihren Komponenten und deren Konzentrationen ist es in der Regel für einen menschlichen Fachmann kaum möglich, all diese Abhängigkeiten in ihrer Gesamtheit mental zu erfassen und gezielt vielversprechende Zusammensetzungen manuell zu spezifizieren. Ein menschlicher Fachmann kann angesichts der enormen Größe des kombinatorischen Möglichkeitsraums von Komponenten und Konzentrationen immer nur einen vergleichsweise kleinen und mehr oder weniger zufällig gewählten Ausschnitt dieses Möglichkeitsraums auch tatsächlich empirisch testen. Es war daher bisher unvermeidlich, viel Zeit und Material auf die Synthese und Analyse von Zusammensetzungen zu verwenden, die letztlich ungewünschte Produkteigenschaften aufwiesen und/oder deren Verwendung als Bestandteil eines Trainingsdatensatzes keine nennenswerte Verbesserung der Qualität eines Vorhersagemodells eines neuronalen Netzwerks bewirkte. Durch die Verwendung eines Active Learning Moduls zur gezielten Auswahl einiger weniger Versuchs-Zusammensetzungen kann der Prozess der Bereitstellung eines geeigneten Trainingsdatensatzes zur Entwicklung eines akkuraten neuronalen Netzwerks erheblich beschleunigt werden.

In einem weiteren vorteilhaften Aspekt kann gemäß Ausführungsformen der Erfindung das System 200 dazu verwendet werden, einen Trainingsdatensatz bereitzustellen, welcher durch gezielte Auswahl von Versuchs-Zusammensetzungen einen historisch bedingten, bestehenden Satz an bekannten Zusammensetzungen in optimaler Weise ergänzt. Bei den bekannten Zusammensetzungen 206 kann es sich um Zusammensetzungen und deren Eigenschaften handeln, die im Laufe des Betriebs eines Labors bzw. einer Laboranlage hergestellt, analysiert und die entsprechenden Daten gespeichert worden sind. Möglicherweise sind die bekannten Zusammensetzungen daher nicht gleichmäßig über den kombinatorischen möglichen Raum aus Komponenten und optional auch Konzentrationen verteilt, sondern ergeben sich zufällig aus der Historie des Betriebs des Labors bzw. der Anlage. Das Active Learning Modul kann dazu verwendet werden und dazu konfiguriert sein, dass prädiktive Modell des neuronalen Netzwerks, dass auf Basis des initialen Trainings auf den bekannten Zusammensetzungen 206 zunächst gebildet wird, mit wenigen weiteren experimentellen Synthesen und Analysen so zu ergänzen, dass zum Beispiel die Komponenten und Konzentrationen, die von den bekannten Zusammensetzungen 206 nur unzureichend abgedeckt sind, nun mittels der gezielt ausgewählten Versuchs-Zusammensetzungen abgedeckt werden.

Nachdem das iterative Training des neuronalen Netzes beendet ist, kann das trainierte Neuronale Netz dazu verwendet werden, Zusammensetzungen ("Prognosezusammensetzungen") vorherzusagen, die bestimmte messtechnisch erfassbare Eigenschaften aufweisen, also sich z. B. im Hinblick auf einen chemischen oder physikalischen oder sonstigen Parameter innerhalb eines definierten und gewünschten Wertebereichs befinden. Hierzu werden die gewünschten Eigenschaften als Eingabe-Vektor präsentiert und in das trainierte neuronale Netz eingegeben. Das neuronale Netz ermittelt daraufhin die Art der Komponenten (und optional auch deren Menge), sowie optional auch die Herstellungsbedingungen, mit denen ein chemisches Erzeugnis mit den gewünschten Eigenschaften erzeugt werden kann. Die Prognosezusammensetzung kann von dem neuronalen Netz automatisch an die Anlage 244 übermittelt werden zusammen mit einem Steuerbefehl, ein chemisches Erzeugnis gemäß der Prognosezusammensetzung zu erzeugen. Optional kann der Steuerbefehl die Anlage auch dazu veranlassen, Eigenschaften dieses Erzeugnisses automatisch zu messen und die Prognosezusammensetzung samt den für diese erhaltenen Eigenschaften in der Datenbank zu speichern und dadurch die Menge bekannter Zusammensetzungen zu erweitern.

Die unterschiedlichen Komponenten des Systems müssen, soweit dies nicht ausdrücklich vermerkt ist, örtlich nicht gebunden sein. Bekanntermaßen können Computersysteme und deren Komponenten weltweit verteilt sein, im Rahmen der Erfindung werden sie jeweils als Einheit betrachtet.

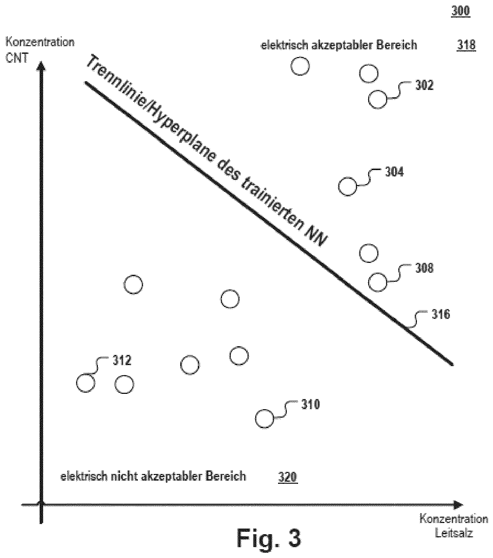

**Figur 3** zeigt einen 2D Ausschnitt eines mehrdimensionalen Datenraums 300, aus welchem das "Active Learning Modul" gezielt Datenpunkte 308 zur Erweiterung des Trainingsdatensatzes auswählt. Im Zuge des Trainings lernt das neuronale Netzwerk, anhand eines Eingabe-Vektors, der die Eigenschaften einer Zusammensetzung spezifiziert, einen Ausgabe-Vektor zu berechnen, welcher eine Zusammensetzung, also eine Spezifikation der Komponenten eines chemischen Erzeugnisses, repräsentiert. Zu den im Eingabevektor spezifizierten Eigenschaften gehören gemäß Ausführungsformen der Erfindung insbesondere die folgenden Eigenschaften sowie Kombinationen von zwei oder mehreren dieser Eigenschaften: Aminoendgruppengehalt, Carboxylendgruppengehalt, , Durchgangwiderstand, Schmelze-Volumenfließrate (MVR), Glasübergangstemperatur, Brennbarkeit (UL94), Kerbschlagzähigkeit, Bruchspannung, Bruchdehnung, Streckspannung E-Modul, Wärmebeständigkeit. Die Kostenreduzierung während der Produktion kann z. B. von einer chemischen Anlage automatisch während der Synthese einer Zusammensetzung erfasst werden und sich beispielsweise auf einen vorgegebenen Referenzwert beziehen. Es ist aber auch möglich, dass ein Mensch die Kosten manuell erfasst. Generell können nur diejenigen Eigenschaften in dem Eingabevektor spezifiziert werden, welche auch Bestandteil des Trainingsdatensatzes waren, auf dem das neuronale Netz trainiert wurde.

Nach dem initialen Training des neuronalen Netzes hat dieses basierend auf den bekannten Zusammensetzungen bereits bestimmte Beziehungen zwischen Komponenten der Zusammensetzungen und einigen Eigenschaften "gelernt". Diese erlernten Beziehungen sind hier durch die Trennlinie 316 veranschaulicht, welche den Datenraum 300 im Hinblick auf die Eigenschaft "Leitfähigkeit" in einen Datenraum 320 mit elektrisch nicht akzeptablen Produkteigenschaften 320 links unterhalb der Trennlinie 316 und einen Datenraum 318 mit elektrisch akzeptablen Produkteigenschaften rechts oberhalb der Trennlinie teilt. Figur 3 kann nur einen auf zwei Dimensionen also entsprechend zwei Komponenten ("Konzentration Leitsalz" und "Konzentration CNT"), eine Eigenschaft und eine Komponente, oder zwei Eigenschaften beschränkten Teilaspekt des Datenraums 300 darstellen. Der Datenraum 300 ist an sich mehrdimensional, er kann z. B. bei 20 Komponenten 190 entsprechende zwiedimensionale Beziehungen (Binominalfuktion: n über 2) haben und jede dieser von diesen 190 Beziehungen gebildeten Räumen enthält bezüglich der jeweils betrachteten Eigenschaft eigene Trennlinien bzw. mehrdimensionale Trennebenen ("Hyperplane").

Die in Figur 3 als Kreise dargestellten Datenpunkte repräsentieren jeweils eine der Versuchs-Zusammensetzungen 208. Die Auswahl einer der Versuchs-Datenpunkte kann gemäß des sog. "Minimum Marginal Hyperplane" Ansatzes erfolgen. Beispielsweise kann das Active Learning Modul als Support-Vector Maschine oder als ein anderer Algorithmus ausgebildet sein, welcher dazu in der Lage ist, einen von den Versuchs-Zusammensetzungen aufgespannten Datenraum im Hinblick auf eine oder mehrere Eigenschaften (oder Komponenten) in Teilräume aufzuteilen auf Basis des bereits vom neuronalen Netzwerk 226 erlernten prädiktiven Modells. Das bereits erlernte Modell des neuronalen Netzwerks ist hier also durch die Trennlinie bzw. Trennebene 316 repräsentiert. Die "Minimum Marginal Hyperplane" Methode geht davon aus, dass die Datenpunkte mit dem kleinsten Abstand zur Trennlinie 316 diejenigen sind, bei denen das bereits erlernte prädiktive Modell, repräsentiert eben durch diese Trennlinie 316, am unsichersten ist und daher die zu diesem Datenpunkt gehörende Versuchs-Zusammensetzung ausgewählt, hergestellt und analysiert werden sollten, um den tatsächliche Eigenschaften, hier z. B. die elektrische Leitfähigkeit, empirisch zu bestimmen. In dem hier dargestellten Beispiel würde das Active-Learning-Modul unter alleiniger Berücksichtigung der Eigenschaft "elektrische Leitfähigkeit" also die von dem Datenpunkt 308 repräsentierte Versuchs-Zusammensetzung auswählen. Die chemische Anlage 244 würde veranlasst werden diese Zusammensetzung (repräsentiert durch Datenpunkt 308) zu synthetisieren und zu analysieren, um die Trainingsdaten mit den Komponenten dieser Versuchs-Zusammensetzung und deren empirisch gemessenen Eigenschaften zu erweitern und das neuronale Netz durch Trainieren auf dem erweiterten Trainingsdatensatz zu verbessern. Beispielweise kann es sein, dass die empirische Messung des gemäß der von Punkt 308 repräsentierten Zusammensetzung ergibt, dass deren elektrische Leitfähigkeit im nicht akzeptablen Bereich 320 liegt. Das Neutrainieren auf dem erweiterten Trainingsdatensatz hätte also zur Folge, dass sich das prädiktive Modell des neuronalen Netzes, hier grafisch visualisiert durch die Trennlinie 316, so anpasst, dass für eine Zusammensetzung wie die durch Punkt 308 repräsentierte in Zukunft die Vorhersage lautet, dass deren elektrische Leitfähigkeit im Bereich 320 liegt. Es würde also die Trennlinie/Trennebene 316 durch das erneute Trainieren auf dem erweiterten Trainingsdatensatz so modifiziert, dass die Linie bzw. Ebene eine "Ausbuchtung" nach rechts oben erhält, sodass das verbesserte neuronale Netz nun erkannt und vorhergesagt hätte, dass die durch Punkt 308 repräsentierte Zusammensetzung im elektrisch nicht akzeptablen Bereich 320 liegt. In der Praxis werden bei der Auswahl des Datenpunkts bzw. der entsprechenden Versuchs-Zusammensetzung vorzugsweise der Abstand der entsprechenden Datenpunkten zu den Trennlinien mehrerer Eigenschaften berücksichtigt, z. B. indem der Datenpunkt mit dem minimalen durchschnittlichen Abstand zu allen Trennlinien/Trennebenen des Datenraums 300 ausgewählt wird.

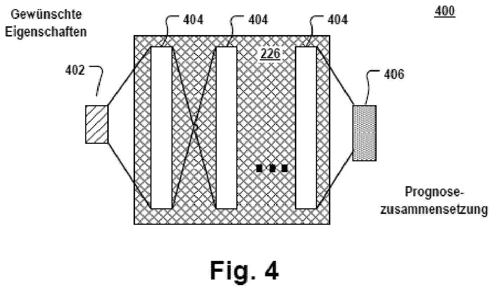

**Figur 4** zeigt die Architektur 400 eines trainierten neuronalen Netzes, das dazu konfiguriert und trainiert ist, einen Eingabe-Vektor 402 als Input zu empfangen und daraus einen Ausgabe-Vektor 406 zu berechnen und auszugeben. Der Eingabe-Vektor 402 spezifiziert die gewünschten Eigenschaften bzw. die entsprechenden Parameterwertbereiche einer Zusammensetzung, deren Komponenten (und optional auch die Konzentrationen oder Mengen der jeweiligen Komponenten) vom neuronalen Netz vorhergesagt (prognostiziert) werden soll. Der Ausgabe-Vektor 406 spezifiziert die Komponenten einer Prognosezusammensetzung und optional auch die Mengen bzw. Konzentrationen dieser Komponenten in der Prognosezusammensetzung, wobei eine Prognosezusammensetzung eine Zusammensetzung ist, von welcher das neuronale Netz vorhersagt, dass deren Eigenschaften innerhalb der im Eingabe-Vektor vorgegebenen Parameterwertbereiche liegt. Das Netzwerk beinhaltet mehrere Schichten 404 von Neuronen, die mit den Neuronen anderer Schichten mittels gewichteter mathematischer Funktionen so verknüpft sind, dass das Netz anhand der im Eingabevektor spezifizierten gewünschten Eigenschaften die Komponenten der entsprechenden Zusammensetzung berechnen, also vorhersagen, kann und diese Komponenten und damit die Prognosezusammensetzung selbst in der Form eines Ausgabevektors 406 ausgeben kann.

Die Neuronen des neuronalen Netzes sind vor dem Training zunächst mit vorgegebenen oder zufälligen Aktivierungen (Gewichte) initialisiert. Das Netzwerk empfängt während des Trainings einen Eingabevektor, der empirisch gemessene Eigenschaften einer bekannten Zusammensetzung repräsentiert, berechnet den Ausgangsvektor mit vorhergesagten Komponenten (und optional Komponentmengen) dieser Zusammensetzung und wird von der Loss-Funktion für Abweichungen der vorhergesagten Komponenten von den tatsächlich verwendeten Komponenten bestraft. Der ermittelte Vorhersagefehler wird über einen Backpropagation genannten Prozess auf die jeweiligen Neuronen zurück verteilt, die ihn verursacht haben, und bewirkt, dass sich die Aktivierungen (Gewichte) bestimmter Neuronen so verändern, dass der Vorhersagefehler (und damit der Wert der Loss-Funktion) verringert. Mathematisch gesehen kann hierfür die Steigung der Loss-Funktion bestimmt werden, sodass die Aktivierungen der Neuronen gerichtet so modifiziert werden können, dass der von der Loss-Funktion ausgegebene Wert minimiert wird. Sobald der Vorhersagefehler bzw. der Loss-Funktionswert unterhalb eines vordefinierten Schwellenwerts liegt, wird das trainierte neuronale Netz als hinreichend präzise angesehen, sodass ein weiteres Training nicht nötig ist.

Beispielsweise kann die Aufgabe sein, eine neue, unbekannte Zusammensetzung zu erzeugen, die eine bestimmte elektrische Leitfähigkeit im Wertebereich EWB, eine bestimmte Farbe im Wertebereich FWB und eine Abriebfestigkeit im Wertebereich AWB aufweist. Bevor diese im Labor real hergestellt wird, soll das neuronale Netzwerk zunächst verwendet werden, um automatisch die Komponenten einer Zusammensetzung zu ermitteln, die die Komponenten eines chemischen Erzeugnisses spezifiziert, deren elektrische Leitfähigkeit, Farbe und Abriebfestigkeit innerhalb der gewünschten Wertebereiche EWB, FWB und AWB liegen. Falls keine Prognosezusammensetzung gefunden wird, deren Eigenschaften innerhalb der gewünschten Wertbereiche liegt, kann auf die Synthese von vorneherein verzichtet und Kosten gespart werden. Hier kann es sinnvoll sein, die Vorgaben im Hinblick auf die Eigenschaften zu ändern.

Die Komponenten dieser neuen Zusammensetzung und optional auch deren jeweilige gewünschte Konzentration werden als Ausgabevektor 406 des neuronalen Netzes an einen Nutzer zur manuellen Auswertung und/oder an eine chemische Anlage ausgegeben. Der Ausgangsvektor kann z. B. 20 Komponenten einer Prognosezusammensetzung beinhalten, von welcher das neuronale Netz prognostizierte, dass diese bzw. das gemäß der Prognosezusammensetzung hergestellte chemische Erzeugnis die gewünschten Eigenschaften aufweist. Bei den eingegebenen Eigenschaften handelt es sich um Eigenschaften, die auch schon beim Training des neuronalen Netzes berücksichtigt wurden. Je nach Art der Zusammensetzung bzw. der als relevant erachteten Eigenschaften können die Vektoren 402, 406 in anderen Ausführungsformen auch eine höhere oder niedrigere Anzahl an Elementen umfassen.

Das erfindungsgemäße Verfahren zur Herstellung einer thermoplastischen Zusammensetzung für mechanisch und/oder thermisch belastete Bauteile umfasst auch die Ausführung nach der die Rezeptur- und Herstellungsanweisung wie vorstehend beschrieben erstellt wurde und die großtechnische Herstellung auf einer anderen Anlage als der vorbeschriebenen Anlage erfolgt. Unter dem Begriff großtechnisch werden Ausführungen verstanden, bei denen als Polymer 10 kg, bevorzugt 50 kg, mehr bevorzugt 100 kg, insbesondere bevorzugt 500 kg, oder mehr verarbeitet werden.

### Liste der Referenznummern

- 102-118: Schritte
- 200: verteiltes System
- 202: DBMS
- 204: Datenbank
- 206: bekannte Zusammensetzungen mit Eigenschaften
- 208: Versuchs-Zusammensetzungen (Eigenschaften unbekannt)
- 210: ursprünglich verwendeter Trainingsdatensatz
- 212: ausgewählte Versuchs-Zusammensetzung
- 214: Auswahlbefehl bezüglich einer Versuchs-Zusammensetzung
- 218: empirisch ermittelte Eigenschaften der ausgewählten Versuchs-Zusammensetzung
- 222: Aktiv Learning Modul
- 224: Computersystem
- 226: neuronales Netz
- 228: Loss-Funktion
- 244: chemische Anlage
- 246: Hauptsteuerungsrechner
- 248: Steuersoftware
- 252: Analysegerät
- 254: Synthesegerät
- 256: Synthesegerät
- 258: Transporteinheit
- 300: 2D Ausschnitt eines Multi-Parameter Datenraums der Versuchs-Zusammensetzungen
- 302-312: Datenpunkte (repräsentiert jeweils eine Versuchs-Zusammensetzung)
- 316: Trennlinie des prädiktiven Modells des trainierten neuronalen Netzwerks
- 318: rheologisch akzeptabler Bereich
- 320: rheologisch nicht-akzeptabler Bereich
- 400: Architektur des neuronalen Netzwerks
- 402: Eingabe-Vektor
- 404: Schichten des neuronalen Netzwerks
- 406: Ausgabe-Vektor

## Patentansprüche

1. Verfahren zur Generierung einer thermoplastischen Zusammensetzung für mechanisch und/oder thermisch belastete Bauteile,
wobei die Komponenten der Zusammensetzung ein oder mehrere Polymere aufweisen wie Polyamide, Polyester, Polyolefine, Polycarbonate und Polyaryletherketone,
wobei die Zusammensetzung durch ein Computersystem (224) generiert wird, wobei das Computersystem auf eine Datenbank (204) Zugriff hat, wobei in der Datenbank bekannte Zusammensetzungen (206), mit deren Komponenten und Eigenschaften gespeichert sind, und wobei das Computersystem mit einer Anlage (244) zur Herstellung und Prüfung von Zusammensetzungen für mechanisch und/oder thermisch belastete Bauteile verbunden ist, wobei das Computersystem ein Neuronales Netzwerk (226) und ein Active Learning Modul (222) umfasst,
mit den Schritten:
a. Verwendung (102) von in der Datenbank gespeicherten bekannten Zusammensetzungen (206) zum Trainieren des neuronalen Netzwerks (226), wobei zum Training eine Loss-Funktion (228) minimiert wird,
b. Prüfung (104), ob der Wert der Loss-Funktion ein vorgegebenes Kriterium erfüllt, wobei selektiv für den Fall, dass das Kriterium nicht erfüllt ist, die folgenden Schritte durchgeführt werden:
i. Auswahl (106) einer Versuchs-Zusammensetzung (212) aus einer Menge vorgegebener Versuchs-Zusammensetzungen (208) durch das Active Learning Modul (222),
ii. Ansteuerung (108) der Anlage (244) durch das Computersystem zur Herstellung und Prüfung der ausgewählten Versuchs-Zusammensetzung,
iii. Trainieren (110) des neuronalen Netzwerks unter Verwendung der ausgewählten Versuchs-Zusammensetzung (212) und deren von der Anlage erfassten Eigenschaften (218),
iv. Wiederholte Durchführung (112) von Schritt b.
c. Generierung (116) einer Prognosezusammensetzung (406) für mechanisch und/oder thermisch belastete Bauteile durch Eingabe eines Eingabevektors (402) in das neuronale Netzwerk (226),
d. Ausgabe (118) der Prognosezusammensetzung (406) durch das neuronale Netzwerk (226).

2. Verfahren gemäß Anspruch 1, wobei die Menge der Versuchs-Zusammensetzungen (208) durch ein Versuchsplanungsprogramm automatisch erzeugt wird.

3. Verfahren gemäß einem der vorigen Ansprüche, wobei neben den Polymeren weitere Komponenten ausgewählt sind aus der Gruppe bestehend aus Verarbeitungshilfsmittel; Nukleierungsmittel und Entformungshilfen, Stabilisatoren; Farbmittel; Leitfähigkeitshilfsmittel (elektrisch); thermische Leitfähigkeitsadditive; Verstärkungsmittel; Schlagzähmodifikatoren; Füllstoffe; Flammschutzmittel und Weichmacher.

4. Verfahren gemäß einem der vorigen Ansprüche, wobei die Eigenschaften ausgewählt sind aus der Gruppe bestehend aus Kerbschlagzähigkeit, Berstumfangsspannung, BET-Oberfläche, Aminoendgruppengehalt, Carboxylendgruppengehalt, Dichte, Dielektrizitätszahl, Konventionelle Durchbiegung, Versagensart, Streckspannung, Durchgangwiderstand, Durchstoß-Maximalkraft-, Anzahl Brüche - Fallhammer- und Kugelfalltests, Farbzahl L*ab, Auszugskraft (Fittings), Korrigierte inhärente Viskosität (CIV), Permeation (Zeit), Schmelze-Volumenfließrate (MVR), Sauerstoff-Index, Oberflächenwiderstand, Schmelzeviskosität, Endgruppensumme, Schmelztemperatur, Schmelzenthalpie, Glasübergangstemperatur, Kristallinität, Maßänderung, Stampfdichte, Transmission, Brennbarkeit (UL94), Vicat-Erweichungstemperatur, Formbeständigkeitstemperatur und Wassergehalt, Bruchdehnung, Streckspannung E-Modul, Wärmebeständigkeit.

5. Verfahren gemäß einem der vorigen Ansprüche, wobei die Ausgabe der Prognosezusammensetzung auf einem Nutzerinterface des Computersystems erfolgt.

6. Verfahren gemäß einem der vorigen Ansprüche, wobei die Anlage (244) mindestens zwei Bearbeitungsstationen (252, 254, 256) aufweist, wobei die mindestens zwei Bearbeitungsstationen über ein Transportsystem (258) miteinander verbunden sind, auf dem Transportvehikel zum Transport der Komponenten der Zusammensetzung und/oder der hergestellten Zusammensetzung zwischen den Bearbeitungsstationen verkehren können, wobei das Verfahren ferner umfasst:
Eingabe einer Zusammensetzung an einen Prozessor, der die Anlage (244) steuert, wobei die in den Prozessor eingegebene Zusammensetzung die ausgewählte Versuchs-Zusammensetzung (212) oder die Prognosezusammensetzung ist, wobei der Prozessor die Anlage ansteuert, die eingegebene Zusammensetzung herzustellen, wobei in den mindestens zwei Bearbeitungsstationen die Herstellung der eingegebenen Zusammensetzung und eine Messung der Eigenschaften der eingegebenen Zusammensetzung erfolgt, wonach eine Ausgabe der gemessenen Eigenschaften auf einem Nutzerinterface der Computersystems erfolgt und/oder die gemessenen Eigenschaften in der Datenbank (204) gespeichert werden.

7. Verfahren gemäß einem der vorigen Ansprüche, wobei das Computersystem (224) über eine Kommunikationsschnittstelle mit der Datenbank (204) und/oder der Anlage (244) zur Herstellung und Prüfung von Zusammensetzungen für mechanisch und/oder thermisch belastete Bauteile kommuniziert, wobei die Kommunikationsschnittstellen ausgewählt sind aus SCSI, USB, FireWire, Bluetooth, Ethernet, WLAN, LAN, Bluetooth oder durch eine andere Netzwerkschnittstelle realisiert werden.

8. Computersystem (224) zur Generierung einer Zusammensetzung für mechanisch und/oder thermisch belastete Bauteile, umfassend eine Datenbank und ein Nutzerinterface, wobei das Computersystem zur Durchführung eines Verfahrens nach einem der Ansprüche 1 bis 7 konfiguriert ist.

9. Computerprogramm, digitales Speichermedium oder Computerprogrammprodukt mit von einem Prozessor ausführbaren Anweisungen, um ein Verfahren nach einem der Ansprüche 1 bis 8 durchzuführen.

10. System (200) umfassend
- eine Anlage (244) zur Herstellung und Prüfung von für mechanisch und/oder thermisch belasteten Bauteilen, wobei die Anlage mindestens zwei Bearbeitungsstationen aufweist, wobei die mindestens zwei Bearbeitungsstationen optional über ein Transportsystem miteinander verbunden sind, auf dem Transportvehikel zum Transport der Komponenten der Zusammensetzung und/oder der hergestellten Zusammensetzung zwischen den Bearbeitungsstationen verkehren können,und
- ein Computersystem (224) gemäß Anspruch 9.

11. Verfahren zur Herstellung einer thermoplastischen Zusammensetzung für mechanisch und/oder thermisch belastete Bauteile, wobei die Rezeptur- und Herstellungsanweisung nach einem der vorhergehenden Ansprüche erstellt wurde und die großtechnische Herstellung auf einer anderen Anlage erfolgt.

## Claims

1. Method for generating a thermoplastic composition for mechanically and/or thermally loaded component parts, wherein the components of the composition include one or more polymers such as polyamides, polyesters, polyolefins, polycarbonates and polyaryletherketones, wherein the composition is generated by a computer system (224), where the computer system has access to a database (204), where known compositions (206) are stored with their components and properties in the database, and where the computer system is connected to a facility (244) for producing and testing compositions for mechanically and/or thermally loaded component parts, wherein the computer system comprises a neural network (226) and an active learning module (222),
comprising the following steps:
a. use (102) of known compositions (206) stored in the database for training the neural network (226), where a loss function (228) is minimized for the training,
b. testing (104) to determine whether the value of the loss function meets a specified criterion,
where selectively, in the event that the criterion is not met, the following steps are carried out:
i. selection (106) of a trial composition (212) from a quantity of specified trial compositions (208) by the active learning module (222),
ii. driving (108) of the facility (244) by the computer system for producing and testing the selected trial composition,
iii. training (110) of the neural network using the selected trial composition (212) and the properties (218) thereof captured by the facility,
iv. repeated implementation (112) of step b,
c. generation (116) of a forecast composition (406) for mechanically and/or thermally loaded component parts by input of an input vector (402) into the neural network (226),
d. output (118) of the forecast composition (406) by the neural network (226).

2. Method according to Claim 1, wherein the quantity of the trial compositions (208) is generated automatically by a trial planning program.

3. Method according to either of the preceding claims, wherein in addition to the polymers further components are selected from the group consisting of processing assistants; nucleating agents and demoulding aids, stabilizers; colourants; conductivity assistants (electrical); thermal conductivity additives; reinforcing agents; impact modifiers; fillers; flame retardants and plasticizers.

4. Method according to any of the preceding claims, wherein the properties are selected from the group consisting of notched impact toughness, bursting periphery tension, BET surface area, amino end-group content, carboxyl end-group content, density, dielectric constant, conventional bowing, failure mode, yield stress, breakdown resistance, maximum penetration force, number of fractures - ball drop and hammer drop tests, colour number L*ab, pull-out force (fittings), corrected intrinsic viscosity (CIV), permeation (time), melt-volume flow rate (MVR), oxygen index, surface resistance, melt viscosity, end-group total, melting temperature, enthalpy of fusion, glass transition temperature, crystallinity, dimensional change, tapped density, transmittance, combustibility (UL94), Vicat softening temperature, heat distortion resistance temperature and water content, elongation at break, yield stress elasticity modulus, heat resistance.

5. Method according to any of the preceding claims, wherein the forecast composition is output on a user interface of the computer system.

6. Method according to any of the preceding claims, wherein the facility (244) comprises at least two workstations (252, 254, 256), where the at least two workstations are connected to one another via a transport system (258) on which transport vehicles are able to run for transporting the components of the composition and/or the composition produced between the workstations, wherein the method further comprises:
input of a composition to a processor which controls the facility (244), where the composition input into the processor is the selected trial composition (212) or the forecast composition, where the processor drives the facility to produce the input composition, where in the at least two workstations the input composition is produced and the properties of the input composition are measured, after which the measured properties are output on a user interface of the computer system and/or the measured properties are stored in the database (204).

7. Method according to any of the preceding claims, wherein the computer system (224) communicates via a communications interface with the database (204) and/or with the facility (244) for producing and testing compositions for mechanically and/or thermally loaded component parts, where the communications interfaces are selected from SCSI, USB, FireWire, Bluetooth, Ethernet, WLAN, LAN, Bluetooth or are realized by another network interface.

8. Computer system (224) for generating a composition for mechanically and/or thermally loaded component parts, comprising a database and a user interface, where the computer system is configured for implementing a method according to any of Claims 1 to 7.

9. Computer program, digital storage medium or computer program product with instructions which can be executed by a processor in order to implement a method according to any of Claims 1 to 8.

10. System (200) comprising
- a facility (244) for producing and testing mechanically and/or thermally loaded component parts, where the facility comprises at least two workstations, where the at least two workstations are connected to one another optionally via a transport system on which transport vehicles are able to run for transporting the components of the composition and/or the composition produced between the workstations, and
- a computer system (224) according to Claim 9.

11. Method for producing a thermoplastic composition for mechanically and/or thermally loaded component parts, wherein the formulation and production instructions have been drawn up as in any of the preceding claims and the industrial production takes place on a different facility.

## Revendications

1. Procédé de génération d'une composition thermoplastique pour des composants à charge mécanique et/ou thermique,
dans lequel les composants de la composition comprennent un ou plusieurs polymères tels que des polyamides, des polyesters, des polyoléfines, des polycarbonates et des polyaryléthercétones,
dans lequel la composition est générée par un système informatique (224), le système informatique ayant accès à une banque de données (204), des compositions connues (206) étant stockées avec leurs composants et propriétés dans la banque de données, et le système informatique étant relié à une installation (244) destinée à fabriquer et vérifier des compositions pour composants à charge mécanique et/ou thermique,
dans lequel le système informatique comprend un réseau neuronal (226) et un module d'apprentissage actif (222),
comprenant les étapes suivantes :
a. utilisation (102) de compositions connues (206) stockées dans la banque de données pour apprentissage du réseau neuronal (226), une fonction de perte (228) étant minimisée pour l'apprentissage,
b. vérification (104) si la valeur de la fonction de perte satisfait un critère prédéfini, les étapes suivantes étant mises en œuvre d'une manière sélective dans le cas dans lequel le critère n'est pas rempli :
i. sélection (106) d'une composition d'essai (212) à partir d'une quantité de compositions d'essai prédéfinies (208) par le module d'apprentissage actif (222),
ii. activation (108) de l'installation (244) par le système informatique de fabrication et de vérification de la composition d'essai sélectionnée,
iii. apprentissage (110) du réseau neuronal par utilisation de la composition d'essai sélectionnée (212) et de ses propriétés (218) déterminées par l'installation,
iv. mise en œuvre répétée (112) de l'étape b,
c. génération (116) d'une composition de pronostic (406) pour des composants à charge mécanique et/ou thermique par entrée d'un vecteur d'entrée (402) dans le réseau neuronal (226),
d. sortie (118) de la composition de pronostic (406) par le réseau neuronal (226).

2. Procédé selon la revendication 1, dans lequel la quantité des compositions d'essai (208) est produite automatiquement par un programme de planification d'essais.

3. Procédé selon l'une des revendications précédentes, dans lequel, outre les polymères, d'autres composants sont sélectionnés dans le groupe consistant en les auxiliaires de mise en oeuvre, les agents de nucléation et les auxiliaires de démoulage, les stabilisants, les colorants, les auxiliaires de conductivité (électrique) ; les additifs de conductivité thermique ; les agents de renforcement ; les modifiants chocs ; les charges ; les retardateurs de flamme et les plastifiants.

4. Procédé selon l'une des revendications précédentes, dans lequel les propriétés sont choisies dans le groupe consistant en la résistance au choc sur barreau entaillé, la contrainte périphérique à l'éclatement, l'aire BET, la teneur en groupes terminaux amino, la teneur en groupes terminaux carboxyle, la masse volumique, la permittivité relative, le fléchissement conventionnel, le type de défaillance, la résistance à la traction, la résistivité volumique, les essais de force maximale de pénétration, de nombre de rupture, au marteau pilon et à la bille tombante, la couleur L*ab, la force d'extraction (raccords), la viscosité intrinsèque corrigée (CIV), la perméation (temps), l'indice de fluidité volumique (MVR), l'indice d'oxygène, la résistance superficielle, la viscosité à l'état fondu, la somme des groupes terminaux, la température de fusion, l'enthalpie de fusion, la température de transition vitreuse, la cristallinité, la déformation, la masse volumique apparente après tassement, le facteur de transmission, l'inflammabilité (UL94), la température de ramollissement de Vicat, la stabilité dimensionnelle à chaud et la teneur en eau, l'allongement à la rupture, la résistance à la traction, le module d'élasticité, la résistance à la chaleur.

5. Procédé selon l'une des revendications précédentes, dans lequel la sortie de la composition de pronostic a lieu sur une interface utilisateur du système informatique.

6. Procédé selon l'une des revendications précédentes, dans lequel l'installation (244) comprend au moins deux postes d'usinage (252, 254, 256), les au moins deux postes d'usinage étant reliés l'un à l'autre par un système de transport (258) sur lequel peuvent se déplacer entre les postes d'usinage des véhicules de transport pour le transport des composants de la composition et/ou de la composition fabriquée, le procédé comprenant en outre :
l'entrée d'une composition dans un processeur, qui commande l'installation (244), la composition entrée dans le processeur étant la composition d'essai sélectionnée (212) ou la composition de pronostic, le processeur commandant l'installation pour fabriquer la composition entrée, la fabrication de la composition entrée et une mesure des propriétés de la composition entrée ayant lieu dans les au moins deux postes d'usinage, ce après quoi a lieu une sortie des propriétés mesurées sur une interface utilisateur du système informatique, et/ou les propriétés mesurées sont stockées dans la banque de données (204).

7. Procédé selon l'une des revendications précédentes, dans lequel le système informatique (224) communique par l'intermédiaire d'une interface de communication avec la banque de données (204) et/ou avec l'installation (244) pour fabriquer et vérifier des compositions pour des composants à charge mécanique et/ou thermique, les intervalles de communication étant choisis parmi SCSI, USB, FireWire, Bluetooth, Ethernet, WLAN, LAN, Bluetooth, ou étant réalisés par une autre interface réseau.

8. Système informatique (224) pour générer une composition pour composants à charge mécanique et/ou thermique, comprenant une banque de données et une interface utilisateur, le système informatique étant configuré pour mettre en œuvre un procédé selon l'une des revendications 1 à 7.

9. Programme informatique, support de stockage numérique ou progiciel informatique comportant des instructions exécutables par un processeur, pour mettre en œuvre un procédé selon l'une des revendications 1 à 8 .

10. Système (200) comprenant
- une installation (244) pour fabriquer et vérifier des composants à charge mécanique et/ou thermique, l'installation comportant au moins deux postes d'usinage, les au moins deux postes d'usinage étant éventuellement reliés l'un à l'autre par un système de transport sur lequel peuvent se déplacer entre les postes d'usinage des véhicules de transport pour le transport des composants de la composition et/ou de la composition fabriquée, et
- un système informatique (224) selon la revendication 9.

11. Procédé de fabrication d'une composition thermoplastique pour des composants à charge mécanique et/ou thermique, dans lequel les instructions de réception et de fabrication ont été établies selon l'une des revendications précédentes, la fabrication à grande échelle étant réalisée sur une autre installation.
